# EUROPEAN PATENT APPLICATION

(11) **EP 4 431 498 A1**
(43) Date of publication of application: **18.09.2024**
(21) Application number: 22890496.7
(22) Date of filing: 04.01.2022
(51) Int. Cl.: C07D 271/113, A61K 31/4245, A61K 31/495, A61K 45/06, A61P 25/28, A61P 35/00, C07D 413/10

(54) **NOVEL OXADIAZOLE DERIVATIVE AND USE THEREOF**

(30) Priority: 08.11.2021 KR 20210152376
(71) Applicant: Celros Biotech, Seoul 03760 (KR)
(72) Inventor: BAE, Yun Soo, Gyeonggi-do 10402 (KR); JEONG, Da Un, Seoul 05070 (KR)
(74) Representative: Altmann Stößel Dick Patentanwälte PartG mbB
(86) International application number: PCT/KR2022/095004
(87) International publication number: WO 2023/080765

(57) **Abstract**

The present disclosure pertains to an oxadiazole derivative and a composition containing same for preventing or treating NADPH oxidase (NOX)-related diseases. The present disclosure can be used to treat NADPH oxidase (NOX)-related diseases through an excellent NOX inhibitory effect.

## Description

### [Technical Field]

The present disclosure relates to a novel oxadiazole derivative and use thereof.

### [Background Art]

NADPH oxidase (NOX) is a family of enzymes with six trans-membrane domains that transfer electrons across biological membranes. These enzymes have been implicated in a variety of pathogenesis through their broad and specific regulation of redox-sensitive signaling pathways. In general, the electron acceptor is oxygen, and a product of the electron transfer reaction is superoxide. Therefore, the main biological function of NOX enzyme is the production of reactive oxygen species (ROS) from oxygen. ROS are small molecules derived from oxygen and include oxygen radicals (super-oxide anions [*O₂], hydroxyl [HO*], peroxyl [ROO*], alkoxyl [RO*], and hydroperoxyl [HOO*]), as well as other oxidants and/or non-radical compounds that are readily converted to radicals such as hydrogen peroxide (H₂O₂).

Several diseases of the central nervous system, including Parkinson's disease, Alzheimer's disease, and the like, commonly involve oxidative stress, inflammation, microglial activation, and progressive neuronal death in the onset and progression of the disease. The expression of NOX1, NOX2, and NOX4 is increased in the brains of Parkinson's disease patients, Alzheimer's disease patients, and amyotrophic lateral sclerosis patients, and neuroprotective effects have been reported when NOX is knocked out or genetically inactivated in disease-induced experimental animals (Parkinson's animal model caused by herbicides, LPS, MPP+, etc., Alzheimer's animal model with APP overexpression, and SOD1 mutant amyotrophic lateral sclerosis animal model). In addition, NOX is known to play an important role in cancer growth, angiogenesis, and metastasis. It has been reported that NOX is overexpressed in epithelial cancers, particularly colorectal, prostate, and breast cancers, and it is associated with the active form of K-Ras. Further, anticancer effects of substances that inhibit NOX have been confirmed in animal experiments.

In particular, an important consideration in the relationship between these diseases and NADPH oxidase is that even though NADPH oxidase is a major source of cellular ROS generation along with mitochondria, the ROS generated by NADPH oxidase is a single product, not a by-product of enzymatic reactions. This suggests that NADPH oxidase plays an important role in cellular function.

Against this backdrop, research on NADPH oxidase has seen a significant increase in recent years, along with a corresponding rise in related research and development funding in the United States, particularly for NOX. However, the progress in research and development of novel, potent NOX inhibitors has been considerably slower, and despite its promise as a disease target, the results to date have been quite limited.

In response to the above problems, the present inventors have identified novel oxadiazole derivatives as NOX inhibitors with excellent activity. In particular, the present inventors found that this NOX inhibition has preventive and/or therapeutic effects on neurodegenerative diseases such as Parkinson's disease and Alzheimer's disease, cancers including various brain carcinomas, and various diseases associated with inhibition of angiogenesis, and completed The present disclosure.

### [Disclosure]

### [Technical Problem]

An object of The present disclosure is to provide a novel oxadiazole derivative or a pharmaceutically acceptable salt thereof.

Another object of The present disclosure is to provide use of the novel oxadiazole derivative or the pharmaceutically acceptable salt thereof.

### [Technical Solution]

### Compounds represented by Chemical Formula 1

In one general aspect, there is provided a compound represented by the following Chemical Formula 1, or a pharmaceutically acceptable salt thereof:

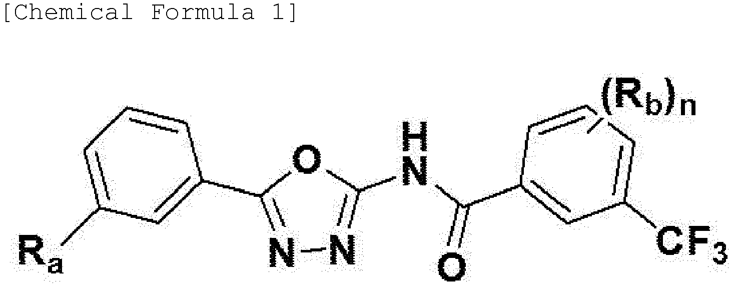

in Chemical Formula 1 above,
Rₐ is hydrogen, halogen, -CF₃, or a 5-6 membered heteroaryl containing at least one heteroatom selected from O, S, and N,
R_{b} is a halogen, and
n is any integer of 1 or 2.

The halogen is F, Cl, Br, or I.

In The present disclosure, the 5-6 membered heteroaryl containing at least one heteroatom selected from O, S and N may be at least any one selected from the group consisting of furanyl, thienyl, pyrrolyl, parazinyl, pyridazinyl, imidazolyl, pyrazolyl, pyrimidinyl, oxazolyl and pyridinyl.

More specifically, the compound represented by Chemical Formula 1 above may be a compound represented by the following Chemical Formula 2 or a pharmaceutically acceptable salt thereof: 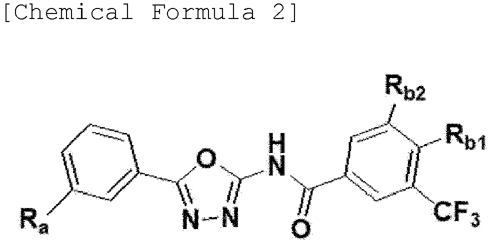 in Chemical Formula 2 above,
Rₐ is hydrogen, halogen, -CF₃, or a 5-6 membered heteroaryl containing at least one heteroatom selected from O, S, and N,
R_{b1} is hydrogen or halogen,
R_{b2} is hydrogen or halogen, and
at least any one of R_{b1} or R_{b2} is a halogen.

More specifically, the compound represented by Chemical Formula 1 above may be a compound represented by the following Chemical Formula 3-1 or a pharmaceutically acceptable salt thereof:

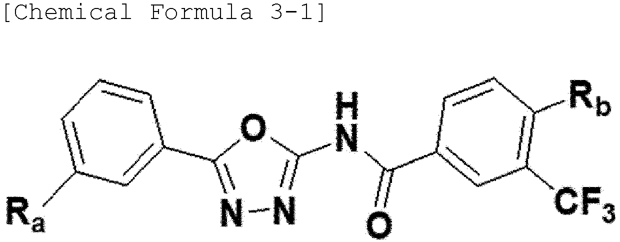

in Chemical Formula 3-1 above,
Rₐ is hydrogen, halogen, -CF₃, or a 5-6 membered heteroaryl containing at least one heteroatom selected from O, S, and N, and
R_{b} is a halogen.

Preferably, Rₐ may be hydrogen, Br, Cl, -CF₃, furanyl or thienyl, and R_{b} may be F or I.

More specifically, the compound represented by Chemical Formula 1 above may be a compound represented by the following Chemical Formula 3-2 or a pharmaceutically acceptable salt thereof:

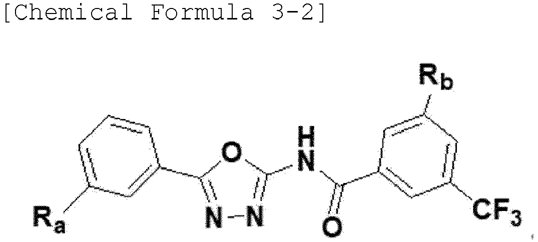

in Chemical Formula 3-2 above,
Rₐ is hydrogen, halogen, -CF₃, or a 5-6 membered heteroaryl containing at least one heteroatom selected from O, S, and N, and
R_{b} is a halogen.

Preferably, Rₐ may be hydrogen, Br, Cl, -CF₃, furanyl or thienyl, and R_{b} may be F.

More specifically, the compound represented by Chemical Formula 1 above may be a compound represented by the following Chemical Formula 4-1 or a pharmaceutically acceptable salt thereof: 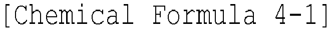 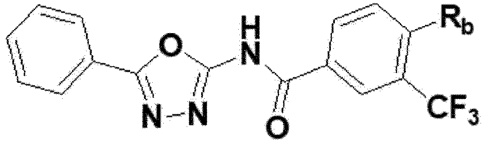 in Chemical Formula 4-1 above,
R_{b} is a halogen.

More specifically, the compound represented by Chemical Formula 1 above may be a compound represented by the following Chemical Formula 4-2 or a pharmaceutically acceptable salt thereof:

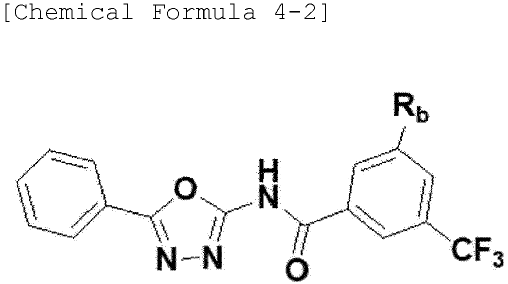

in Chemical Formula 4-2 above,
R_{b} is a halogen.

In another embodiment, preferably, the compound represented by Chemical Formula 1 above may be a compound in which Rₐ is F, Cl, Br, I, CF₃, furanyl, thienyl, or pyrrolyl, R_{b} is F, Cl, Br, or I, and n is 1.

In another embodiment, preferably, the compound represented by Chemical Formula 1 above may be a compound in which Rₐ is hydrogen, R_{b} is F, Cl, Br or I, and n is 1.

In another embodiment, preferably, the compound represented by Chemical Formula 1 above may be a compound in which Rₐ is Cl, Br, or CF₃, R_{b} is F, and n is 1.

In another embodiment, preferably, the compound represented by Chemical Formula 1 above may be a compound in which Rₐ is furanyl, thienyl, or pyrrolyl, R_{b} is F, and n is 1.

In another embodiment, preferably, the compound represented by Chemical Formula 1 above may be a compound in which Rₐ is Br or Cl, R_{b} is F, and n is 1.

In another embodiment, preferably, the compound represented by Chemical Formula 1 above may be a compound in which Rₐ is hydrogen, R_{b} is F or I, and n is 1.

In another embodiment, preferably, the compound represented by Chemical Formula 1 above may be a compound in which Rₐ is CF₃, R_{b} is F, and n is 1.

In another embodiment, preferably, the compound represented by Chemical Formula 1 above may be a compound in which Rₐ is furanyl, R_{b} is F, and n is 1.

In another embodiment, the compound represented by Chemical Formula 1 above may be a compound in which Rₐ is thienyl, R_{b} is F, and n is 1.

In another embodiment, preferably, the compound represented by Chemical Formula 2 above may be a compound in which Rₐ is F, Cl, Br, I, or CF₃, R_{b1} is F, Cl, Br, or I, and R_{b2} is hydrogen.

In another embodiment, preferably, the compound represented by Chemical Formula 2 above may be a compound in which Rₐ is F, Cl, Br, I, or CF₃, R_{b1} is hydrogen, and R_{b2} is F, Cl, Br or I.

In another embodiment, preferably, the compound represented by Chemical Formula 2 above may be a compound in which Rₐ is hydrogen, R_{b1} is F, Cl, Br, or I, and R_{b2} is hydrogen.

In another embodiment, preferably, the compound represented by Chemical Formula 2 above may be a compound in which Rₐ is hydrogen, R_{b1} is hydrogen, and R_{b2} is F, Cl, Br, or I.

In another embodiment, preferably, the compound represented by Chemical Formula 2 above may be a compound in which Rₐ is furanyl, thienyl, or pyrrolyl, R_{b1} is F, Cl, Br, or I, and R_{b2} is hydrogen.

In another embodiment, preferably, the compound represented by Chemical Formula 2 above may be a compound in which Rₐ is furanyl, thienyl, or pyrrolyl, R_{b1} is hydrogen, and R_{b2} is F, Cl, Br, or I.

In another embodiment, preferably, the compound represented by Chemical Formula 2 above may be a compound in which Rₐ is hydrogen, F, Cl, Br, I, or CF₃, R_{b1} is F or I, and R_{b2} is hydrogen.

In another embodiment, preferably, the compound represented by Chemical Formula 2 above may be a compound in which Rₐ is hydrogen, F, Cl, Br, I, or CF₃, R_{b1} is hydrogen, and R_{b2} is F.

In another embodiment, preferably, the compound represented by Chemical Formula 2 above may be a compound in which Rₐ is furanyl or thienyl, R_{b1} is F, and R_{b2} is hydrogen.

In another embodiment, preferably, the compound represented by Chemical Formula 2 above may be a compound in which Rₐ is furanyl or thienyl, R_{b1} is hydrogen, and R_{b2} is F.

The compound represented by Chemical Formula 1 may be any one selected from the group consisting of the following compounds:
*N*-(5-(3-chlorophenyl)-1,3,4-oxadiazol-2-yl)-3-fluoro-5-(trifluoromethyl)benzamide;
4-fluoro-*N*-(5-phenyl-1,3,4-oxadiazol-2-yl)-3-(trifluoromethyl)benzamide;
3-fluoro-*N*-(5-phenyl-1,3,4-oxadiazol-2-yl)-5-(trifluoromethyl)benzamide;
*N*-(5-(3-bromophenyl)-1,3,4-oxadiazol-2-yl)-4-fluoro-3-(trifluoromethyl)benzamide;
4-fluoro-3-(trifluoromethyl)-*N*-(5-(3-(trifluoromethyl)phenyl)-1,3,4-oxadiazol-2-yl)benzamide;
*N*-(5-(3-chlorophenyl)-1,3,4-oxadiazol-2-yl)-4-fluoro-3-(trifluoromethyl)benzamide;
4-iodo-*N*-(5-phenyl-1,3,4-oxadiazol-2-yl)-3-(trifluoromethyl)benzamide;
4-fluoro-*N*-(5-(3-thiophen-3-yl)phenyl)-1,3,4-oxadiazol-2-yl)-3-(trifluoromethyl)benzamide;
3-fluoro-*N*-(5-(3-thiophen-3-yl)phenyl)-1,3,4-oxadiazol-2-yl)-5-(trifluoromethyl)benzamide;
4-fluoro-*N*-(5-(3-furan-3-yl)phenyl)-1,3,4-oxadiazol-2-yl)-3-(trifluoromethyl)benzamide;
3-fluoro-*N*-(5-(3-furan-3-yl)phenyl)-1,3,4-oxadiazol-2-yl)-5-(trifluoromethyl)benzamide;
3-fluoro-5-(trifluoromethyl)-*N*-(5-(3-(trifluoromethyl)phenyl)-1,3,4-oxadiazol-2-yl)benzamide; and
*N*-(5-(3-bromophenyl)-1,3,4-oxadiazol-2-yl)-3-fluoro-5-(trifluoromethyl)benzamide.

The compound represented by Chemical Formula 1 may be any one selected from the group consisting of the following compounds:

| **Example No.** | **Structure** | **Example No.** | **Structure** |
|---|---|---|---|
| **1** | 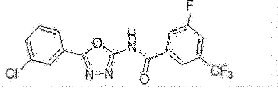 | **2** | 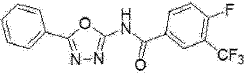 |
| **3** | 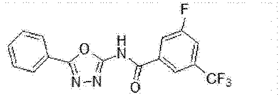 | **4** | 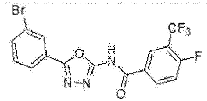 |
| **5** | 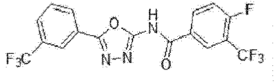 | **6** | 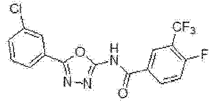 |
| **7** | 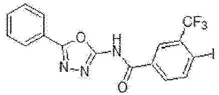 | **8** | 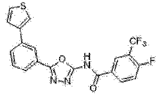 |
| **9** | 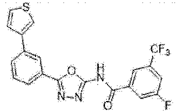 | **10** | 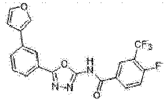 |
| **11** | 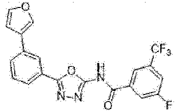 | **12** | 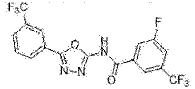 |
| **13** | 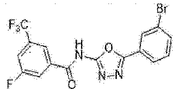 | | |

In The present disclosure, the pharmaceutically acceptable salt means a salt commonly used in the pharmaceutical industry, and may include, for example, inorganic ion salts prepared from calcium, potassium, sodium, magnesium, and the like, inorganic acid salts prepared from hydrochloric acid, nitric acid, phosphoric acid, bromic acid, iodic acid, perchloric acid, and sulfuric acid, and the like; organic acid salts prepared from acetic acid, trifluoroacetic acid, citric acid, maleic acid, succinic acid, oxalic acid, benzoic acid, tartaric acid, fumaric acid, manderic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like; sulfonic acid salts prepared from methanesulfonic acid, ethanesulfonic acid, benzenesulfonic acid, p-toluenesulfonic acid, naphthalenesulfonic acid, and the like; amino acid salts prepared from glycine, arginine, lysine, and the like; and amine salts prepared with trimethylamine, triethylamine, ammonia, pyridine, picoline, and the like, but the types of salts referred to in The present disclosure are not limited by these listed salts.

### Use of compound represented by Chemical Formula 1

The present disclosure provides use of a compound represented by the following Chemical Formula 1 or a pharmaceutically acceptable salt thereof:

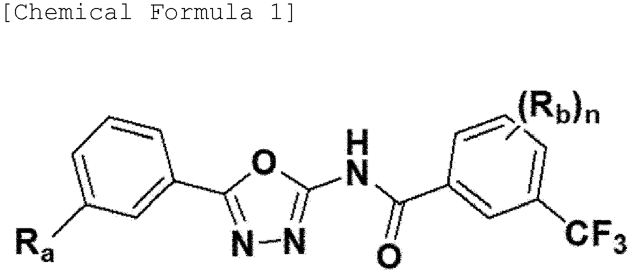

in Chemical Formula 1 above,
Rₐ is hydrogen, halogen, -CF₃, or a 5-6 membered heteroaryl containing at least one heteroatom selected from O, S, and N,
R_{b} is a halogen, and
n is any integer of 1 or 2.

In The present disclosure, matters regarding the Chemical Formula 1 above may be applied to the above-described matters regarding Chemical Formulas 2, 3-1, 3-2, 4-1, 4-2, and specific compounds thereof within the scope of use of The present disclosure.

The present disclosure provides a pharmaceutical composition comprising: the compound represented by Chemical Formula 1 above or a pharmaceutically acceptable salt thereof.

The present disclosure provides a pharmaceutical composition comprising: the compound represented by Chemical Formula 1 above or a pharmaceutically acceptable salt thereof; and a pharmaceutically acceptable carrier.

The present disclosure provides a NADPH oxidase (NOX) inhibitor comprising: the compound represented by Chemical Formula 1 above or a pharmaceutically acceptable salt thereof.

The compounds according to The present disclosure exhibit excellent effects in inhibiting NOX (NADPH oxidase), which may be utilized in various therapeutic uses.

The present disclosure provides a pharmaceutical composition for preventing or treating NADPH oxidase (NOX)-related diseases, comprising: the compound represented by Chemical Formula 1 above or a pharmaceutically acceptable salt thereof.

Members of the NADPH oxidase (NOX) family are enzymes that produce reactive oxygen species (ROS) as primary product thereof. These enzymes typically reduce oxygen molecules in a NADPH-dependent manner in order to produce superoxide anions. This mechanism of regulation of reactive oxygen species production through the active control of NOX is ultimately closely related to overall cell signaling systems and is therefore highly relevant not only to overall cell signaling systems but also to the treatment of the pathogenesis of various diseases.

For example, many diseases of the central nervous system associated with neurodegenerative brain disorders, including Parkinson's disease, Alzheimer's disease, and the like, commonly exhibit oxidative stress and neuroinflammation in the development and progression of pathogenesis thereof. In particular, NOX has been identified as a top regulator of both oxidative stress and neuroinflammation. Therefore, inactivation or pharmacological inhibition of NOX may have potent neuroprotective effects (e.g., protection of dopaminergic neurons, amelioration of microglial pathology, etc.) and behavioral improvements in a wide range of neurological diseases.

In addition, for example, overexpression of NOX is well documented in various cancers, especially brain carcinomas, and inhibition thereof may be highly effective in treating the diseases.

Reactive oxygen species generated by NOX are pathophysiologically relevant in cancer, as they are associated with altered signal transduction, an environment related to tumor formation and growth, angiogenesis, gene instability, and metastasis.

Since the types of NOX enzymes that are mainly expressed depending on the organ and cell are different, many NOX isoenzyme-specific studies have been conducted in various cancers. NOX1 has been reported to be associated with colon cancer proliferation, while NOX2 is associated with breast cancer, colorectal cancer, gastric cancer, prostate cancer, and myelomonocytic leukemia, etc. NOX4 has been linked to non-small cell lung cancer, gastric cancer, and neuroblastoma; NOX5 to prostate cancer, breast cancer, melanoma, and lung cancer; and Duox1 to lung and liver cancer.

In addition, NOX inhibitors may have anti-tumor effects, restore sensitivity to immunotherapy, and/or improve response to immunotherapy. Further, NOX inhibitors may have anti-angiogenic effects. They may also inhibit the expression of inflammatory factors.

Accordingly, NADPH oxidase (NOX)-related diseases may comprise, for example, psoriasis, rheumatoid arthritis, osteoarthritis, atherosclerosis, ulcers, liver cirrhosis, glomerulonephritis, diabetic nephropathy, malignant nephrosclerosis, thrombotic microangiopathy, organ transplant rejection, diabetes, hypertension, cardiac hypertrophy, heart failure, restenosis, cancer, autoimmune diseases, inflammatory diseases, degenerative brain diseases, retinal diseases, and the like.

The autoimmune diseases include alopecia areata, ankylosing spondylitis, antiphospholipid syndrome, autoimmune diseases of adrenal glands, autoimmune hemolytic anemia, autoimmune hepatitis, autoimmune oophoritis, autoimmune orchitis, autoimmune thrombocytopenia, Behçet's disease, bullous pemphigoid, cardiomyopathy, chronic fatigue immune dysfunction syndrome, chronic inflammatory demyelinating polyneuropathy, cicatricial pemphigoid, CREST syndrome, cold agglutinin disease, Crohn's disease, discoid lupus, Graves' disease, Guillain-Barré syndrome, Hashimoto's thyroiditis, idiopathic pulmonary fibrosis, idiopathic thrombocytopenic purpura, IgA neuritis, juvenile arthritis, lichen planus, Meniere's disease, mixed connective tissue disease, multiple sclerosis, type I or immune-mediated diabetes mellitus, myasthenia gravis, pemphigus vulgaris, pernicious anemia, and crystalline polyarteritis nodosa, polychondritis, autoimmune polyglandular syndrome, rheumatic polymyalgia rheumatica, polymyositis and dermatomyositis, primary agammaglobulinemia, primary biliary cirrhosis, systemic lupus erythematosus, lupus erythematosus, Tagayasu's arteritis, transient arteritis, giant cell arteritis, ulcerative colitis, uveitis, vitiligo, and Wegener's granulomatosis, but are not limited thereto.

Examples of inflammatory diseases capable of being prevented or treated by the composition of The present disclosure include asthma, encephalitis, inflammatory enteritis, chronic obstructive pulmonary disease, allergy, septic shock, pulmonary fibrosis, undifferentiated spondyloarthropathy, undifferentiated arthropathy, arthritis, inflammatory osteolysis, and chronic inflammation due to chronic viral or bacterial infection, but the inflammatory diseases are not limited thereto.

According to a preferred embodiment of The present disclosure, examples of cancers capable of being prevented or treated by the composition of The present disclosure include brain cancer, neuroendocrine cancer, gastric cancer, lung cancer, breast cancer, ovarian cancer, liver cancer, bronchial cancer, nasopharyngeal cancer, laryngeal cancer, pancreatic cancer, bladder cancer, adrenal cancer, colorectal cancer, colon cancer, cervical cancer, prostate cancer, bone cancer, skin cancer, thyroid cancer, parathyroid cancer and ureter cancer, but the cancer is not limited thereto.

The cancer may be, in particular, brain cancer. The brain cancer may be, for example, at least any one selected from the group consisting of astrocytoma, glioblastoma, ependymoma, oligodendroglioma, mixed glioma, brain stem glioma, optic nerve glioma, pituitary adenoma, craniopharyngioma, medulloblastoma, primitive neuroectodermal tumors, pineal tumors, meningioma, schwannoma, metastatic brain tumors, CNS lymphoma, neurofibromatosis, pseudotumor cerebri, and tuberous sclerosis.

More specifically, the compound represented by Chemical Formula 1 according to The present disclosure or a pharmaceutically acceptable salt thereof is useful for preventing or treating degenerative brain diseases.

Accordingly, The present disclosure provides a pharmaceutical composition for preventing or treating degenerative brain diseases, comprising: the compound represented by Chemical Formula 1 above or a pharmaceutically acceptable salt thereof.

Non-limiting examples of degenerative brain diseases in The present disclosure may include Parkinson's disease, Huntington's disease, Alzheimer's disease, mild cognitive impairment, senile dementia, amyotrophic lateral sclerosis, spinocerebellar atrophy, Tourette's syndrome, Friedrich's Ataxia, Machado-Joseph's disease, Lewy body dementia, dystonia, progressive supranuclear palsy, and frontotemporal dementia.

More specifically, the compound represented by Chemical Formula 1 according to The present disclosure, or a pharmaceutically acceptable salt thereof is useful for preventing or treating retinal diseases.

Accordingly, The present disclosure provides a pharmaceutical composition for preventing or treating retinal diseases, comprising: the compound represented by Chemical Formula 1, or a pharmaceutically acceptable salt thereof.

Non-limiting examples of retinal diseases in The present disclosure may include glaucoma, retinopathy of prematurity, proliferative retinopathy, corneal transplant rejection, proliferative vitreoretinopathy, diabetic retinopathy, macular degeneration, choroidal neovascularization, retinal edema, and the like. Specifically, the macular degeneration may be wet macular degeneration or dry macular degeneration.

According to an embodiment of The present disclosure, the oxadiazole derivatives according to The present disclosure have a good effect on NOX inhibition. In particular, it is possible to exhibit excellent effects in preventing or treating NADPH oxidase (NOX)-related diseases by modulating the production of NOX-induced reactive oxygen species in various neuronal cell lines, including the microglial cell line BV2.

According to an embodiment of The present disclosure, the oxadiazole derivatives according to The present disclosure may be effective in the treatment of diseases by inhibiting the migratory ability of microglial cell lines.

According to an embodiment of The present disclosure, the oxadiazole derivatives according to The present disclosure exhibit molecular biological and histological improvements in Parkinson's disease models, including protection of dopaminergic neurons and amelioration of microglial pathology, and these effects are accompanied by behavioral therapeutic effects.

According to an embodiment of the invention, the oxadiazole derivatives according to The present disclosure are effective in reducing amyloid plaque deposition, inhibiting the activity of astrocytes and microglia, and improving cognitive function, spatial memory, short-term and long-term memory in Alzheimer's disease models.

According to an embodiment of The present disclosure, the oxadiazole derivatives according to The present disclosure have the advantages of good metabolic stability and high brain barrier blood (BBB) permeability. Accordingly, the oxadiazole derivatives according to The present disclosure have potential for use in the treatment of degenerative brain diseases and/or brain carcinomas where BBB permeation may be important.

According to an embodiment of The present disclosure, the oxadiazole derivatives according to The present disclosure exhibit growth inhibitory and proliferation inhibitory effects on glioblastoma, and also exhibit good anticancer effects in combination with other anticancer drugs.

Accordingly, The present disclosure also provides a pharmaceutical composition for preventing or treating cancer, comprising: the compound represented by Chemical Formula 1 above or a pharmaceutically acceptable salt thereof; and a second anticancer agent.

The second anti-cancer agent may be, for example, any compound known to exhibit anti-cancer effects. For example, the second anti-cancer agent may be any one selected from the group consisting of bifunctional alkylating agents (preferably Cyclophosphamide, Mechlorethamine, Chlorambucil or Melphalan); monofunctional alkylating agents (preferably Dacarbazine (DTIC), Nitrosourea or Temozolomide); Anthracyclines (preferably Daunorubicin, Doxorubicin, Epirubicin, Idarubicin, Mitoxantrone or Valrubicin); Taxanes (preferably Paclitaxel, Docetaxel, Nab-paclitaxel or Taxotere); Epothilone (preferably Patupilone, Sagopilone or Ixabepilone); Deacetylase inhibitors (preferably Vorinostat or Romidepsin); Inhibitors of Topoisomerase I (preferably Irinotecan or Topotecan); Inhibitors of Topoisomerase II (preferably Etoposide, Teniposide or Tafluposide); Kinase inhibitors (preferably Bortezomib, Erlotinib, Gefitinib, Imatinib, Vemurafenib or Vismodegib); Nucleotide analogs and/or precursor analogs (preferably Azacitidine, Azathioprine, Capecitabine, Cytarabine, Doxifluridine, Fluorouracil, Gemcitabine, Hydroxy Urea, Mercaptopurine, Methotrexate or Thioguanine); Peptide antibiotic (preferably Bleomycin or Actinomycin); Platinum-based agents (preferably Carboplatin, Cisplatin or Oxaliplatin); Retinoids (preferably Tretinoin, Alitretinoin or Bexarotene); and Vinca alkaloids and derivatives (preferably Vinblastine, Vincristine, Vindesine or Vinorelbine).

More preferably, it may be at least any one selected from the group consisting of Cyclophosphamide; Melphalan; Docetaxel; Paclitaxel; Nab-paclitaxel; Carboplatin; Cisplatin; Oxaliplatin; Methotrexate; Pemetrexed; Azathioprine; Capecitabine; Fluouracil; Mercaptopurine; Gemcitabine; Bleomycin; Actinomycin; Vincristine; Vinblastine; Vinorelbine; Retinoic acid; Temozolomide; Daunorubicin; Doxorubicin; Irinotecan; and Topotecan.

In the above combination, it may be provided in the form of a combination. Further, it may also be provided for use concurrently, separately, or sequentially in the treatment of cancer.

With respect to combination therapy, it is not important whether the compound represented by Chemical Formula 1 and the second anti-cancer agent according to The present disclosure are administered simultaneously, for example, as a single composition, or sequentially, for example, as two separate compositions. It is essential that an effective amount of the first administered compound/agent remains within the patient's body upon administration of the second compound/agent. The combination of a compound represented by Chemical Formula 1 and the second anti-cancer agent may be formulated for simultaneous administration, separate administration, or sequential administration. In particular, if the administration of the compounds is not simultaneous, the compounds are administered at relatively close times to each other. Further, the compounds may be administered in the same or different dosage forms or by the same or different routes of administration, for example, one compound may be administered topically and the other may be administered orally. The combination of two compounds is administered, for example, - as a combination that is part of the same medicinal formulation (then the two compounds are always administered at the same time);
as a combination of two units/compositions (each containing one of the substances, with the possibility of simultaneous, sequential or separate administration).

The combination treatments according to The present disclosure may be utilized for the treatment through combination of two or more types in a positive manner in that it exhibits a synergistic effect.

In patients with brain tumor treated with temozolomide, lower expression of NOX was associated with increased survival, and in the case of Avastin, increased expression of NOX was associated with increased resistance to the drug. Thus, the expression of NOX appears to be strongly associated with resistance to anticancer drugs, and therefore, combination therapy with NOX inhibitors is needed.

For administration, the pharmaceutical composition of The present disclosure may further comprise at least one pharmaceutically acceptable carrier in addition to the compound represented by Chemical Formula 1 or a pharmaceutically acceptable salt thereof. The pharmaceutically acceptable carrier may be saline, sterile water, Ringer's solution, buffered saline, dextrose solution, maltodextrin solution, glycerol, ethanol, or a mixture of one or more of these components, and, if necessary, may contain other conventional additives such as antioxidants, buffers, bacteriostatic agents, and the like. Further, the composition may be formulated into injectable formulations such as aqueous solutions, suspensions, emulsions, and the like, pills, capsules, granules or tablets by further adding diluents, dispersants, surfactants, binders and lubricants. Accordingly, the pharmaceutical composition of The present disclosure may be a patch, liquid, pill, capsule, granule, tablet, suppository, or the like. These preparations may be prepared by conventional methods used for formulation in the art or methods disclosed in the document [see, Remington's Pharmaceutical Science (latest edition), Mack Publishing Company, Easton PA], and formulated into various preparations depending on respective diseases or components.

The composition of The present disclosure may be administered orally or parenterally (for example, intravenous, subcutaneous, intraperitoneal or topical application) according to the desired method, and the dosage varies depending on the patient's weight, age, sex, health condition, diet, administration time, administration method, excretion rate, and type and severity of the disease. The daily dosage of the compound of The present disclosure is about 0.01 to 1000 mg/kg, preferably 0.1 to 100 mg/kg, and may be divided and administered once or several times a day.

A dosage of the co-administered second anti-cancer agent may be adjusted within an appropriate range known to those skilled in the art, in consideration of the daily dosage of Chemical Formula 1 above.

In other words, the composition may be administered through any general route as long as the composition is able to reach the target tissue, but for example, the composition may be administered through intradermal injection, intravein injection, intraperitoneal injection, intravitreal injection, subcutaneous injection using an osmotic pump, or the like.

The pharmaceutical composition of The present disclosure may further comprise at least one active ingredient exhibiting the same or similar efficacy in addition to the compound represented by Chemical Formula 1 or a pharmaceutically acceptable salt thereof.

The present disclosure provides a method for treating or preventing NADPH oxidase (NOX)-related diseases, comprising: administering, to a subject in need thereof, a therapeutically effective amount of the compound represented by Chemical Formula 1, or a pharmaceutically acceptable salt thereof.

The present disclosure provides a method for treating or preventing degenerative brain diseases, comprising: administering, to a subject in need thereof, a therapeutically effective amount of the compound represented by Chemical Formula 1, or a pharmaceutically acceptable salt thereof.

The present disclosure provides a method for treating or preventing cancer, comprising: administering, to a subject in need thereof, a therapeutically effective amount of the compound represented by Chemical Formula 1, or a pharmaceutically acceptable salt thereof.

The term "therapeutically effective amount" as used herein refers to an amount of the compound represented by Chemical Formula 1, or a pharmaceutically acceptable salt effective for preventing or treating the diseases.

The treatment method of The present disclosure includes not only treating the disease itself prior to the onset of symptoms, but also inhibiting or avoiding symptoms thereof, by administering the compound represented by Chemical Formula 1. In the management of diseases, a prophylactic or therapeutic dose of a particular active ingredient will vary depending on the nature and severity of the disease or condition and the route by which the active ingredient is administered. The dose and frequency of dose will vary depending on the individual patient's age, weight and response. A suitable dosage regimen may be readily selected by those skilled in the art who take these factors for granted. In addition, the treatment method of The present disclosure may further comprise administering a therapeutically effective amount of an additional active agent to aid in the treatment of the disease, in combination with the compound represented by Chemical Formula 1 above, wherein the additional active agent may have a synergistic or adjuvant effect with the compound represented by Chemical Formula 1 above.

The present disclosure provides a food composition for preventing or improving NADPH oxidase (NOX)-related diseases, comprising: the compound represented by Chemical Formula 1, or a pharmaceutically acceptable salt thereof.

The present disclosure provides a food composition for preventing or improving degenerative brain diseases, comprising: the compound represented by Chemical Formula 1, or a pharmaceutically acceptable salt thereof.

The present disclosure provides a food composition for preventing or improving cancer, comprising: the compound represented by Chemical Formula 1, or a pharmaceutically acceptable salt thereof.

The food composition of The present disclosure may be used as a health functional food. The term "health functional food" refers to food manufactured and processed using raw materials or ingredients having useful functionality for human body in accordance with Health Functional Food Act No. 6727, wherein the term "functional" means intake for the purpose of obtaining useful effects for health purposes, such as regulating nutrients or exerting physiological actions, on the structure and function of the human body.

The food composition of The present disclosure may comprise conventional food additives, and the suitability of the "food additives" is judged by specifications and standards for relevant items in accordance with general test methods and general principles of Korean Food Additives Code approved by the Food and Drug Administration, unless there is no other regulation thereto.

For the purpose of preventing and/or improving the diseases, the food composition of The present disclosure may contain the compound represented by Chemical Formula 1 in an amount of 0.01 to 95 wt%, preferably 1 to 80 wt%, based on the total weight of the composition. Further, for the purpose of preventing and/or improving the above diseases, the food composition of The present disclosure may be manufactured and processed in the form of tablets, capsules, powders, granules, liquids, pills, beverages, and the like.

In addition, The present disclosure provides use of the compound represented by Chemical Formula 1, or a pharmaceutically acceptable salt thereof for the manufacture of medicaments for the treatment of NADPH oxidase (NOX)-related diseases.

Further, The present disclosure provides use of the compound represented by Chemical Formula 1, or a pharmaceutically acceptable salt thereof for the manufacture of medicaments for the treatment of degenerative brain diseases.

Further, The present disclosure provides use of the compound represented by Chemical Formula 1, or a pharmaceutically acceptable salt thereof for the manufacture of medicaments for the treatment of cancer.

The compound represented by Chemical Formula 1 for the manufacture of medicaments may be mixed with acceptable adjuvants, diluents, carriers, and the like, and may be prepared as a combined preparation with other active agents to have a synergistic effect of the active ingredients.

Further, The present disclosure provides a food composition for enhancing cognitive function comprising: the compound represented by Chemical Formula 1, or a pharmaceutically acceptable salt thereof.

The present disclosure also includes the following embodiments:
a compound represented by Chemical Formula 1 as defined in any of the embodiments described herein, or a pharmaceutically acceptable salt thereof, for use as a medicament;
a compound represented by Chemical Formula 1 as defined in any of the embodiments described herein, or a pharmaceutically acceptable salt thereof, for use in the prevention or treatment of the diseases discussed herein;
a method for treating the above-described diseases comprising: administering, to a subject in need thereof, a therapeutically effective amount of the compound represented by Chemical Formula 1 as defined in any of the embodiments described herein, or a pharmaceutically acceptable salt thereof;
the compound represented by Chemical Formula 1 as defined in any of the embodiments described herein, or a pharmaceutically acceptable salt thereof, for the manufacture of medicaments for the treatment of the above-described diseases;
a NOX inhibitor comprising: the compound represented by Chemical Formula 1 as defined in any of the embodiments described herein, or a pharmaceutically acceptable salt thereof;
an antioxidant comprising: the compound represented by Chemical Formula 1 as defined in any of the embodiments described herein, or a pharmaceutically acceptable salt thereof; and
a method for treating cancer comprising: administering, to a subject in need thereof, a therapeutically effective amount of the compound represented by Chemical Formula 1 as defined in any of the embodiments described herein, or a pharmaceutically acceptable salt thereof; and a second anti-cancer agent.

All examples, isomers thereof, or pharmaceutically acceptable salts thereof may be claimed individually or as a group together in any combination with any number of each and every embodiment described herein.

Matters described in the composition, use, and treatment method of The present disclosure are equally applied unless they contradict each other.

### [Advantageous Effects]

The compound represented by Chemical Formula 1 according to The present disclosure or a pharmaceutically acceptable salt thereof exhibits excellent NADPH oxidase (NOX) inhibitory effect. In particular, the compound represented by Chemical Formula 1 or a pharmaceutically acceptable salt thereof exhibits excellent effects in the treatment of NOX-related diseases, including degenerative brain diseases, cancer, and the like. In addition, the compound represented by Chemical Formula 1 or a pharmaceutically acceptable salt thereof has excellent metabolic stability and excellent permeability to the blood-brain barrier (BBB), thereby having high potential for application in various diseases.

### [Description of Drawings]

FIG. 1 shows the results of IC₅₀ value measurement to confirm the NOX inhibitory effect of compounds according to The present disclosure.
FIG. 2 shows the results of the NOX inhibition assay according to treatment of a compound of Example 1 in BV2 cells.
FIG. 3 shows the results of the NOX inhibition assay according to the treatment of the compound of Example 1 in BV2 cells.
FIG. 4 shows the results of determining the cytotoxicity of compounds of Examples 1 and 2.
FIG. 5 shows the results of inhibiting microglial migration according to the treatment with the compound of Example 2.
FIG. 6 shows the results of confirming the protective effect on dopaminergic neurons according to the treatment with the compound of Example 1.
FIG. 7 shows the results of confirming the protective effect on dopaminergic neurons according to the treatment with the compound of Example 2.
FIG. 8 shows the results of ameliorating microglial pathology according to the treatment with the compound of Example 1.
FIG. 9 shows the results of ameliorating microglial pathology according to the treatment with the compound of Example 2.
FIG. 10 shows the results of improving motor function according to the treatment with the compound of Example 1.
FIG. 11 shows the results of improving motor function according to the treatment with the compound of Example 2.
FIG. 12 shows the results of inhibiting amyloid plaque deposition according to the treatment with the compound of Example 2.
FIG. 13 shows the results of inhibiting astrocyte activity according to the treatment with the compound of Example 2.
FIG. 14 shows the results of inhibiting microglial activity according to the treatment with the compound of Example 2.
FIG. 15 shows the results of improving cognitive function according to administration of the compound of Example 2 in an experiment to confirm the improvement of cognitive function through novel object recognition and novel location recognition tests.
FIG. 16 shows the results of a water maze test to confirm spatial memory improvement according to administration of the compound of Example 2.
FIG. 17 shows the results of a passive avoidance test to determine short-term and long-term memory improvement according to administration of the compound of Example 2.
FIG. 18 shows the results of the cancer cell growth inhibition effect on glioblastoma in vitro according to treatment with the compound of Example 2.
FIG. 19 shows results of the inhibition of brain tumor growth and enhancement of survival according to treatment with the compound of Example 1 in a mouse animal model xenografted with a brain tumor glioblastoma.
FIG. 20 shows results of the inhibition of brain tumor growth and enhancement of survival according to administration of the compound of Example 2 and coadministration of the compound of Example 2 and Temozolomide in a mouse animal model xenografted with a brain tumor glioblastoma.

### [Best Mode]

Hereinafter, embodiments of The present disclosure will be described in detail so that those skilled in the art are able to easily practice The present disclosure. However, The present disclosure may be implemented in various different forms and is not limited to the embodiments described herein.

Reagents and solvents described below were purchased from Sigma-Aldrich and TCI unless otherwise specified.

¹H- and ¹³C-NMR of all compounds were measured with a Bruker AV-500, and CDCl₃ (d_{H} = 7.26 ppm and d_{C} = 77.0 ppm) was employed as an internal standard. NMR data were obtained using MNova 10.0 processing software (Mestrelab Research).

High-resolution mass spectrometry was performed using a Joel JMS-700 mass spectrometer based on electrical ionization.

### Example 1. Synthesis of N-(5-(3-chlorophenyl)-1,3,4-oxadiazol-2-yl)-3-fluoro-5-(trifluoromethyl)benzamide (LMT-

2219)

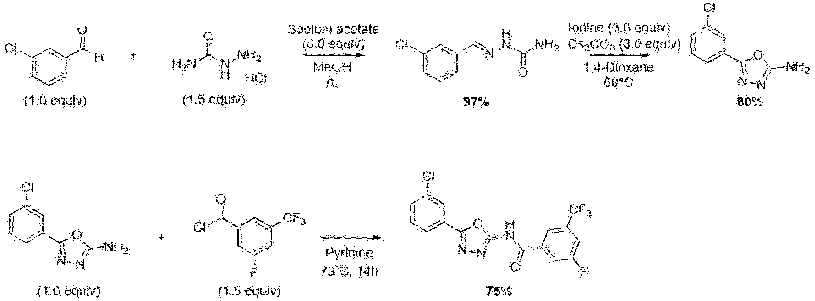

### (1) Synthesis of (E) -2-(3-chlorobenzylidine)hydrazinecarboxamide (1.4 g, 97% yield, white solid):

To a solution of 3-chlorobenzenaldehyde (1.0 g, 7.1 mmol) and sodium acetate (1.8 g, 21.3 mmol) in MeOH (300 mL), semicarbazide hydrochloride (1.2 g, 10.7 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-chlorophenyl)-1,3,4-oxadiazol-2-amine (1.1 g, 80% yield, yellow solid):

(*E*)-2-(3-chlorobenzylidine)hydrazinecarboxamide (1.4 g, 7.08 mmol), Cs₂CO₃ (6.9 g, 21.3 mmol), iodine (5.4 g, 21.3 mmol) was added to 1,4-dioxane (200 mL). The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.; ¹H NMR (500 MHz, DMSO-*d*₆): δ_{H} 7.73-7.71 (m, 2H), 7.56-7.52 (m, 2H), 7.35 (s, 2H) ppm.

### (3) Synthesis of N-(5-(3-chlorophenyl)-1,3,4-oxadiazol-2-yl)-3-fluoro-5-(trifluoromethyl)benzamide (1.6 g, 75% yield, white solid):

To a stirred solution of 5-(3-chlorophenyl)-1,3,4-oxadiazol-2-amine (1.1 g, 5.7 mmol) in pyridine (100 mL), 3-fluoro-5-(trifluoromethyl)benzoylchloride (1.9 g, 8.5 mmol) was added. The reaction mixture was stirred at 73°C for 14 hours and then cooled to room temperature. To the mixture, 10% hydrochloric acid (50 ml) and water (50 ml) were added, followed by extraction with ethyl acetate (40 mL) (x3). The combined organic layers were dried over anhydrous MgSO₄, filtered, and concentrated in vacuo. The residue was purified by filtration using DCM and n-Hexane to obtain the product; ¹H NMR (500 MHz, CDCl₃): δ_{H} 12.67 (s, 1H), 8.26 (s, 1H), 8.15 (d, J = 9.0 Hz, 1H), 8.05 (d, J = 7.9 Hz, 1H), 7.95-7.89 (m, 2H), 7.72 (d, J = 8.0 Hz, 1H), 7.66 (t, J = 8.1 Hz, 1H) ppm; HRMS (EI) *m*/*z* [M]⁺ calcd for C₁₆H₉ClF₄N₃O₂, 386.0, found 386.0.

### Example 2. Synthesis of 4-fluoro-N-(5-phenyl-1,3,4-oxadiazol-2-yl)-3-(trifluoromethyl)benzamide (LMT-2131)

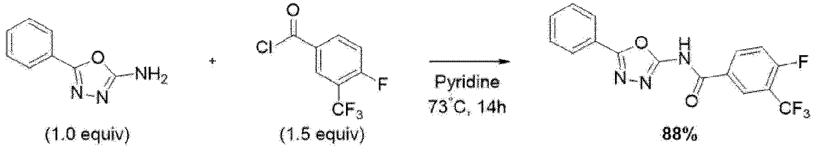

### (1) Synthesis of 4-fluoro-N-(5-phenyl-1,3,4-oxadiazol-2-yl)-3-(trifluoromethyl)benzamide (2.3 g, 88% yield, white solid):

To a solution of 5-phenyl-1,3,4-oxadiazol-2-amine (1.2 g, 7.4 mmol) in pyridine (100 mL), 4-fluoro-3-(trifluoromethyl)benzoylchloride (2.5 g, 11.2 mmol) was added. The reaction mixture was stirred at 73°C for 14 hours and then cooled to room temperature. To the mixture, 10% hydrochloric acid (50 ml) and water (50 ml) were added, followed by extraction with ethyl acetate (40 mL) (x3). The combined organic layers were dried over anhydrous MgSO₄, filtered, and concentrated in vacuo. The residue was purified by filtration using DCM and n-Hexane to obtain the product; ¹H NMR (500 MHz, DMSO-*d*₆): δ_{H} 12.74 (s, 1H), 8.46 (d, *J* = 6.1 Hz, 1H), 8.41 (s, 1H), 7.97 (d, *J* = 6.6Hz, 2H), 7.73 (t, *J* = 9.5Hz, 1H), 7.63-7.61 (m, 3H) ppm. HRMS (EI) *m*/*z* [M]⁺ calcd for C₁₆H₁₀F₄N₃O₂, 352.1, found 352.1.

### Example 3. Synthesis of 3-fluoro-N-(5-phenyl-1,3,4-oxadiazol-2-yl)-5-(trifluoromethyl)benzamide (LMT-2142)

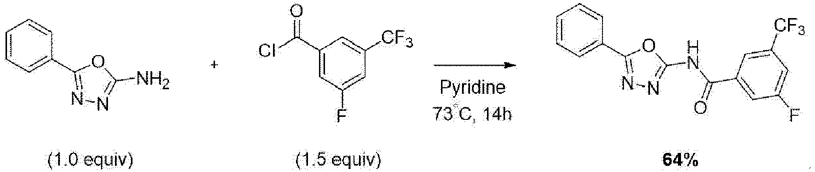

To a solution of 5-phenyl-1,3,4-oxadiazol-2-amine (50 mg, 0.31 mmol) in pyridine (3 mL), 3-fluoro-5-(trifluoromethyl)benzoylchloride (0.07 ml, 0.47 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product (70 mg, 64% yield, white solid).

¹H NMR (500 MHz, DMSO-*d₆*) : δ_{H} 12.58 (s, 1H), 8.27 (s, 1H), 8.16 (d, *J* = 8.8Hz, 1H), 8.05 (d, *J* = 7.7Hz, 1H), 7.98 (d, *J* = 6.8Hz, 2H), 7.67-7.63 (m, 3H) ppm. HRMS (EI) *m*/*z* [M]⁺ calcd for C₁₆H₁₀F₄N₃O₂, 352.1, found 352.1.

### Example 4. Synthesis of N-(5-(3-bromophenyl)-1,3,4-oxadiazol-2-yl)-4-fluoro-3-(trifluoromethyl)benzamide (LMT-2183)

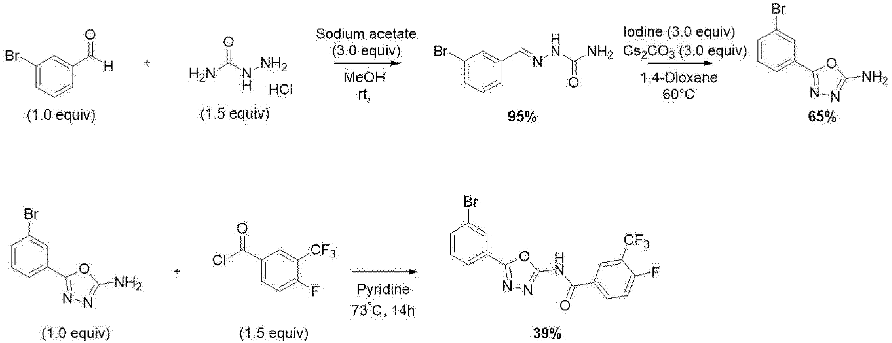

### (1) Synthesis of (E)-2-(3-bromobenzylidine)hydrazinecarboxamide (4.4 g, 90% yield, white solid):

To a solution of 3-bromobenzenaldehyde (1.9 ml, 16.2 mmol) and sodium acetate (2.0 g, 24.3 mmol) in MeOH (300 mL), semicarbazide hydrochloride (5.4 g, 48.6 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-bromophenyl)-1,3,4-oxadiazol-2-amine (2.8 g, 65% yield, yellow solid):

To 1,4-dioxane (200 mL), (E)-2-(3-bromobenzylidine)hydrazinecarboxamide (4.4 g, 18.3 mmol), Cs₂CO₃ (17.9 g, 54.9 mmol), and iodine (13.9 g, 54.9 mmol) were added. The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.

¹H NMR (500 MHz, DMSO-*d₆*) : δ_{H} 7.91 (s, 1H), 7.80 (d, J = 7.8 Hz, 1H), 7.72 (d, J = 8.0 Hz, 1H), 7.51 (t, J = 7.9 Hz, 1H), 7.38 (s, 2H) ppm.

### (3) Synthesis of N-(5-(3-bromophenyl)-1,3,4-oxadiazol-2-yl)-4-fluoro-3-(trifluoromethyl)benzamide (35 mg, 39% yield, white solid):

To a stirred solution of 5-(3-bromophenyl)-1,3,4-oxadiazol-2-amine (50 mg, 0.21 mmol) in pyridine (3 mL), 4-fluoro-3-(trifluoromethyl)benzoylchloride (0.05 ml, 0.32 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d₆*): δ_{H} 12.71 (s, 1H), 8.47 (d, J = 6.5 Hz, 1H), 8.45 - 8.36 (m, 1H), 8.07 (s, 1H), 7.97 (d, *J* = 7.7 Hz, 1H), 7.85 (d, *J* = 8.2 Hz, 1H), 7.75 (t, J = 9.7 Hz, 1H), 7.59 (t, *J* = 7.9 Hz, 1H) ppm. HRMS (EI) *m*/*z* [M]⁺ calcd for C₁₆H₉BrF₄N₃O₂, 430.0, found 386.0.

### Example 5. Synthesis of 4-fluoro-3-(trifluoromethyl)-N-(5-(3-(trifluoromethyl)phenyl)-1,3,4-oxadiazol-2-yl)benzamide (LMT-2206)

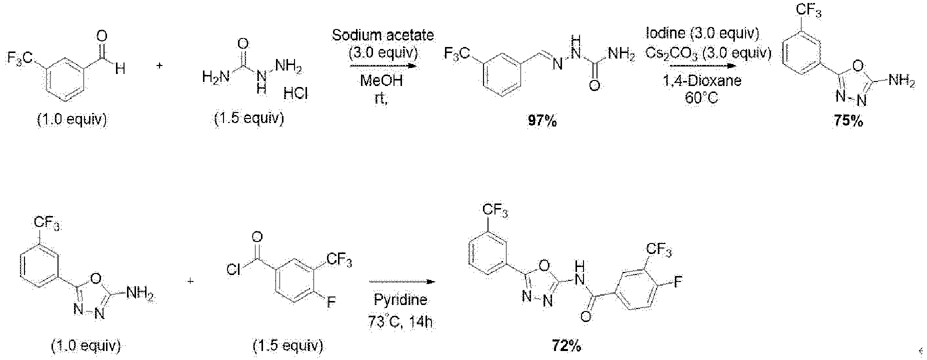

### (1) Synthesis of (E)-2-(3-(trifluoromethyl)benzylidine)hydrazinecarboxamide (1.3 g, 97% yield, white solid):

To a solution of 3-(trifluoromethyl)benzenaldehyde (1 g, 5.7 mmol) and sodium acetate (1.4 g, 17.2 mmol) in MeOH (300 mL), semicarbazide hydrochloride (0.96 g, 8.6 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-(trifluoromethyl)phenyl)-1,3,4-oxadiazol-2-amine (960 mg, 75% yield, white solid):

To 1,4-dioxane (50 mL), (*E*)-2-(3-(trifluoromethyl)benzylidine)hydrazinecarboxamide (1.3 g, 5.6 mmol), Cs₂CO₃ (5.4 g, 16.7 mmol), and iodine (4.2 g, 16.7 mmol) were added. The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.

### (3) Synthesis of 4-fluoro-3-(trifluoromethyl)-N-(5-(3-(trifluoromethyl)phenyl)-1,3,4-oxadiazol-2-yl)benzamide (66 mg, 72% yield, white solid):

To a stirred solution of 5-(3-(trifluoromethyl)phenyl)-1,3,4-oxadiazol-2-amine (50 mg, 0.22 mmol) in pyridine (3 mL), 4-fluoro-3-(trifluoromethyl)benzoylchloride (0.05 ml, 0.33 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d*₆) : δ_{H} 8.47 (d, *J* = 6.2 Hz, 1H), 8.44 - 8.38 (m, 1H), 8.27 (d, *J* = 7.7 Hz, 1H), 8.18 (s, 1H), 8.03 (d, *J* = 7.7 Hz, 1H), 7.88 (t, *J* = 7.7 Hz, 1H), 7.74 (t, *J* = 9.5 Hz, 1H) ppm. HRMS (EI) *m*/*z* [M]⁺ calcd for C₁₇H₉F₇N₃O₂, 420.1, found 386.0.

### Example 6. Synthesis of N-(5-(3-chlorophenyl)-1,3,4-oxadiazol-2-yl)-4-fluoro-3-(trifluoromethyl)benzamide (LMT-2218)

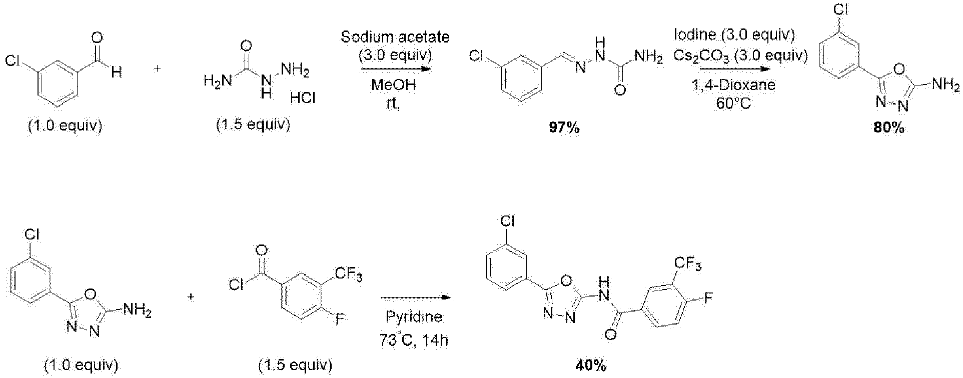

### (1) Synthesis of (E) -2-(3-chlorobenzylidine)hydrazinecarboxamide (1.4 g, 97% yield, white solid):

To a solution of 3-chlorobenzenaldehyde (1.0 g, 7.1 mmol) and sodium acetate (1.8 g, 21.3 mmol) in MeOH (300 mL), semicarbazide hydrochloride (1.2 g, 10.7 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-chlorophenyl)-1,3,4-oxadiazol-2-amine (1.1 g, 80% yield, yellow solid):

(*E*)-2-(3-chlorobenzylidine)hydrazinecarboxamide (1.4 g, 7.08 mmol), Cs₂CO₃ (6.9 g, 21.3 mmol), iodine (5.4 g, 21.3 mmol) was added to 1,4-dioxane (200 mL) . The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.

¹H NMR (500 MHz, DMSO-*d₆*): δ_{H} 7.73-7.71 (m, 2H), 7.56-7.52 (m, 2H), 7.35 (s, 2H) ppm.

### (3) Synthesis of N-(5-(3-chlorophenyl)-1,3,4-oxadiazol-2-yl)-4-fluoro-3-(trifluoromethyl)benzamide (1.6 g, 75% yield, white solid):

To a stirred solution of 5-(3-chlorophenyl)-1,3,4-oxadiazol-2-amine (50 mg, 0.26 mmol) in pyridine (3 mL), 4-fluoro-3-(trifluoromethyl)benzoylchloride (0.06 ml, 0.39 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d*₆): δ_{H} 8.46 (d, *J* = 6.0 Hz, 1H), 8.40 (d, *J* = 5.5 Hz, 1H), 7.92 (d, *J* = 6.7 Hz, 2H), 7.73 (dd, *J* = 16.7, 8.4 Hz, 2H), 7.65 (t, *J* = 7.8 Hz, 1H) ppm. HRMS (EI) *m*/*z* [M]⁺ calcd for C₁₆H₉ClF₄N₃O₂, 386.0, found 386.0.

### Example 7. Synthesis of 4-iodo-N-(5-phenyl-1,3,4-oxadiazol-2-yl)-3-(trifluoromethyl)benzamide (LMT-2240)

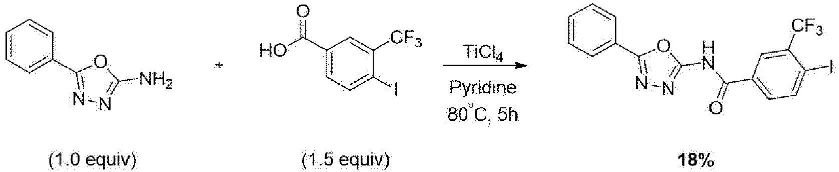

To a solution of 5-phenyl-1,3,4-oxadiazol-2-amine (500 mg, 3.1 mmol) in pyridine (10 mL), 4-iodo-3-(trifluoromethyl)benzoic acid (1200 mg, 4.0 mmol) and TiCl₄ (1M in DCM) (9.3 ml, 9.3 mmol) were added. The reaction mixture was stirred at 80°C for 5 hours and then cooled to room temperature. To the mixture, 10% hydrochloric acid (30 ml) and water (30 ml) were added, followed by extraction with ethyl acetate (60 mL) (x3). The combined organic layers were dried over anhydrous MgSO₄, filtered, and concentrated in vacuo. The residue was purified by filtration using DCM and n-Hexane to obtain the product (250 mg, 18% yield, white solid).

¹H NMR (500 MHz, DMSO-*d*₆) : δ_{H} 8.38 (s, 1H), 8.36 (d, *J* = 8.2 Hz, 1H), 8.01 (d, *J* = 9.4 Hz, 1H), 7.98 (d, *J* = 6.4 Hz, 2H), 7.68 - 7.59 (m, 3H) ppm. HRMS (EI) *m*/*z* [M]⁺ calcd for C₁₆H₁₀F₃IN₃O₂, 460.0, found 352.1.

### Example 8. Synthesis of 4-fluoro-N-(5-(3-thiophen-3-yl)phenyl)-1,3,4-oxadiazol-2-yl)-3-(trifluoromethyl)benzamide (LMT-2187)

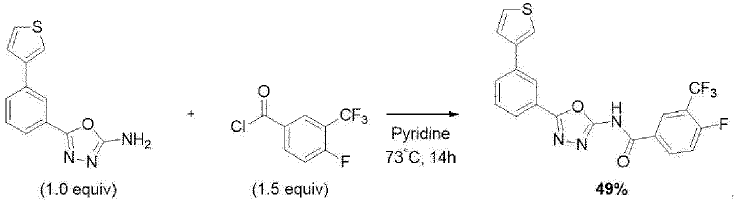

### (1) Synthesis of 4-fluoro-N-(5-(3-thiophen-3-yl)phenyl)-1,3,4-oxadiazol-2-yl)-3-(trifluoromethyl)benzamide (45 mg, 49% yield, brown solid):

To a stirred solution of 5-(3-(thiophen-3-yl)phenyl)-1,3,4-oxadiazol-2-amine (50 mg, 0.21 mmol) in pyridine (3 mL), 4-fluoro-3-(trifluoromethyl)benzoylchloride (0.04 ml, 0.27 mmol) was stirred for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d*₆) : δ_{H} 12.56 (s, 1H), 8.49 (d, *J* = 6.5 Hz, 1H), 8.43 (s, 1H), 8.24 (s, 1H), 8.06 (s, 1H), 7.99 (d, *J* = 7.8 Hz, 1H), 7.89 (d, *J* = 7.8 Hz, 1H), 7.76 (d, *J* = 9.6 Hz, 1H), 7.74 - 7.70 (m, 1H), 7.68 (d, *J* = 7.8 Hz, 1H), 7.65 (d, *J* = 5.5 Hz, 1H) ppm. HRMS (EI) *m*/*z* [M]⁺ calcd for C₂₀H₁₂F₄N₃O₂S, 434.1, found 386.0.

### Example 9. Synthesis of 3-fluoro-N-(5-(3-thiophen-3-yl)phenyl)-1,3,4-oxadiazol-2-yl)-5-(trifluoromethyl)benzamide (LMT-2188)

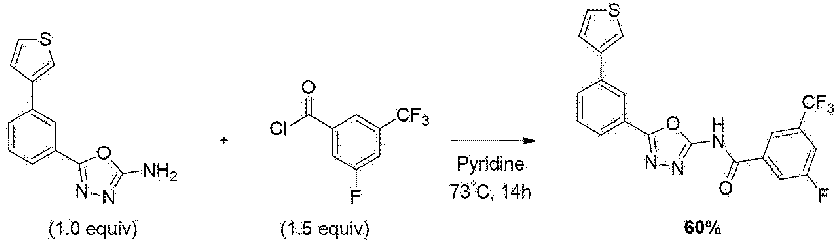

To a stirred solution of 5-(3-(thiophen-3-yl)phenyl)-1,3,4-oxadiazol-2-amine (50 mg, 0.21 mmol) in pyridine (3 mL), 3-fluoro-5-(trifluoromethyl)benzoylchloride (0.04 ml, 0.27 mmol) was stirred for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product (55 mg, 60% yield, brown solid).

¹H NMR (500 MHz, DMSO-*d*₆) : δ_{H} 12.79 (s, 1H), 8.27 (s, 1H), 8.23 (s, 1H), 8.15 (d, *J* = 8.3 Hz, 1H), 8.05 (s, 2H), 7.98 (d, *J* = 7.3 Hz, 1H), 7.88 (d, *J* = 7.4 Hz, 1H), 7.71 (s, 1H), 7.69 - 7.59 (m, 2H) ppm. HRMS (EI) *m*/*z* [M]⁺ calcd for C₂₀H₁₂F₄N₃O₂S, 434.1, found 386.0.

### Example 10. Synthesis of 4-fluoro-N-(5-(3-furan-3-yl)phenyl)-1,3,4-oxadiazol-2-yl)-3-(trifluoromethyl)benzamide (LMT-2189)

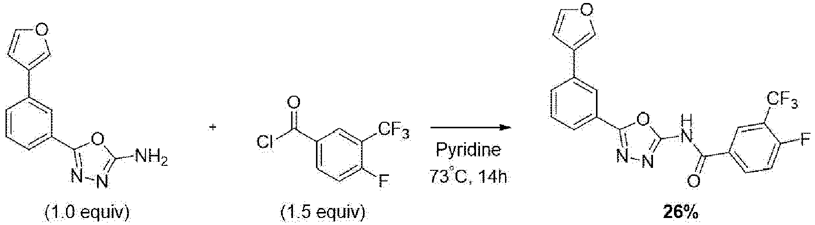

To a stirred solution of 5-(3-(furan-3-yl)phenyl)-1,3,4-oxadiazol-2-amine (50 mg, 0.22 mmol) in pyridine (3 mL), 4-fluoro-3-(trifluoromethyl)benzoylchloride (0.04 ml, 0.29 mmol) was stirred for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product (24 mg, 26% yield, brown solid).

¹H NMR (500 MHz, DMSO-*d*₆): δ_{H} 8.47 (d, *J* = 6.3 Hz, 1H), 8.41 (s, 1H), 8.35 (s, 1H), 8.15 (s, 1H), 7.88 (d, *J* = 7.7 Hz, 1H), 7.84 (d, *J* = 7.6 Hz, 1H), 7.80 (s, 1H), 7.73 (t, J = 9.6 Hz, 1H), 7.63 (t, *J* = 7.7 Hz, 1H), 7.06 (s, 1H) ppm. HRMS (EI) *m*/*z* [M]⁺ calcd for C₂₀H₁₂F₄N₃O₃, 418.1, found 386.0.

### Example 11. Synthesis of 3-fluoro-N-(5-(3-furan-3-yl)phenyl)-1,3,4-oxadiazol-2-yl)-5-(trifluoromethyl)benzamide (LMT-2190)

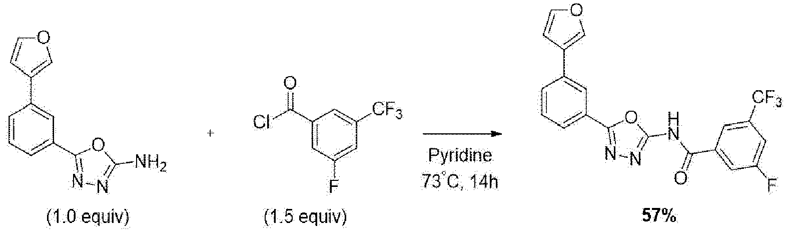

To a stirred solution of 5-(3-(furan-3-yl)phenyl)-1,3,4-oxadiazol-2-amine (50 mg, 0.22 mmol) in pyridine (3 mL), 3-fluoro-5-(trifluoromethyl)benzoylchloride (0.04 ml, 0.29 mmol) was stirred for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product (52 mg, 57% yield, brown solid).

¹H NMR (500 MHz, DMSO-*d*₆) : δ_{H} 12.76 (s, 1H), 8.37 (s, 1H), 8.28 (s, 1H), 8.16 (s, 2H), 8.05 (d, *J* = 8.0 Hz, 1H), 7.89 (d, *J* = 7.7 Hz, 1H), 7.85 (d, *J* = 7.7 Hz, 1H), 7.82 (s, 1H), 7.64 (t, *J* = 7.8 Hz, 1H), 7.08 (s, 1H) ppm. HRMS (EI) *m*/*z* [M]⁺ calcd for C₂₀H₁₂F₄N₃O₃, 418.1, found 386.0.

### Example 12. Synthesis of 3-fluoro-5-(trifluoromethyl)-N-(5-(3-(trifluoromethyl)phenyl)-1,3,4-oxadiazol-2-yl)benzamide (LMT-2207)

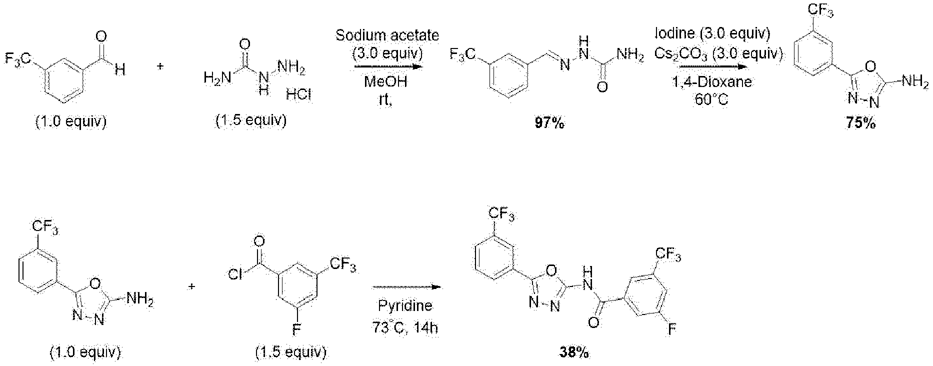

### (1) Synthesis of (E)-2-(3-(trifluoromethyl)benzylidine)hydrazinecarboxamide (1.3 g, 97% yield, white solid):

To a solution of 3-(trifluoromethyl)benzenaldehyde (1 g, 5.7 mmol) and sodium acetate (1.4 g, 17.2 mmol) in MeOH (300 mL), semicarbazide hydrochloride (0.96 g, 8.6 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-(trifluoromethyl)phenyl)-1,3,4-oxadiazol-2-amine (960 mg, 75% yield, white solid):

To 1,4-dioxane (50 mL), (*E*)-2-(3-(trifluoromethyl)benzylidine)hydrazinecarboxamide (1.3 g, 5.6 mmol), Cs₂CO₃ (5.4 g, 16.7 mmol), and iodine (4.2 g, 16.7 mmol) were added. The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.

### (3) Synthesis of 3-fluoro-5-(trifluoromethyl)-N-(5-(3-(trifluoromethyl)phenyl)-1,3,4-oxadiazol-2-yl)benzamide (35 mg, 38% yield, white solid):

To a stirred solution of 5-(3-(trifluoromethyl)phenyl)-1,3,4-oxadiazol-2-amine (50 mg, 0.22 mmol) in pyridine (3 mL), 3-fluoro-5-(trifluoromethyl)benzoylchloride (0.05 ml, 0.33 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d*₆) : δ_{H} 8.27 (s, 2H), 8.22 - 8.12 (m, 2H), 8.09 - 7.99 (m, 2H), 7.89 (t, *J* = 7.8 Hz, 1H) ppm. HRMS (EI) *m*/*z* [M]⁺ calcd for C₁₇H₉F₇N₃O₂, 420.1, found 386.0.

### Example 13. Synthesis of N-(5-(3-bromophenyl)-1,3,4-oxadiazol-2-yl)-3-fluoro-5-(trifluoromethyl)benzamide (LMT-2184)

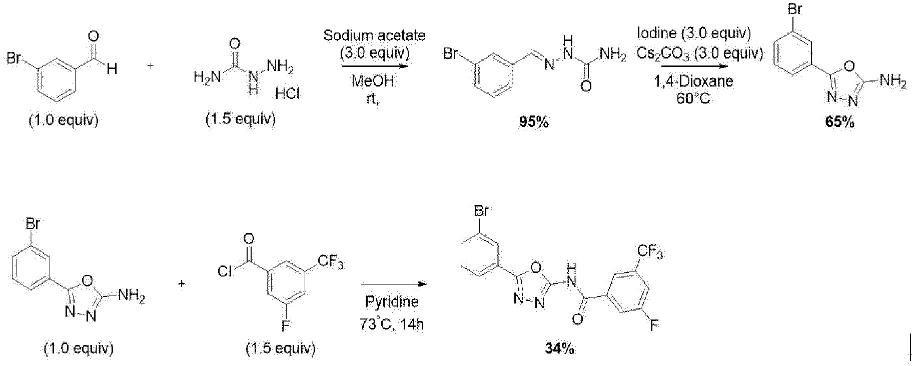

### (1) Synthesis of (E)-2-(3-bromobenzylidine)hydrazinecarboxamide (4.4 g, 90% yield, white solid):

To a solution of 3-bromobenzenaldehyde (1.9 ml, 16.2 mmol) and sodium acetate (2.0 g, 24.3 mmol) in MeOH (300 mL), semicarbazide hydrochloride (5.4 g, 48.6 mmol) was added. The reaction mixture was stirred at room temperature for 30 minutes. The mixture was concentrated in vacuo, and water (100 mL) was added, followed by extraction with ethyl acetate (150 mL) (x3). The combined organic layers were filtered and concentrated in vacuo. The residue was purified by filtration and used in the next reaction.

### (2) Synthesis of 5-(3-bromophenyl)-1,3,4-oxadiazol-2-amine (2.8 g, 65% yield, yellow solid):

To 1,4-dioxane (200 mL), (*E*)-2-(3-bromobenzylidine)hydrazinecarboxamide (4.4 g, 18.3 mmol), Cs₂CO₃ (17.9 g, 54.9 mmol), and iodine (13.9 g, 54.9 mmol) were added. The reaction mixture was stirred at 75°C for 30 minutes. The mixture was allowed to cool to room temperature, and sodium thiosulfate (250 mL) was added, followed by extraction with ethyl acetate (150 mL) (x3). The combined organic layers were dried over anhydrous MgSO₄, filtered, and concentrated in vacuo. The residue was purified by filtration using diethyl ether to obtain the product.

¹H NMR (500 MHz, DMSO-*d*₆) : δ_{H} 7.91 (s, 1H), 7.80 (d, J = 7.8 Hz, 1H), 7.72 (d, J = 8.0 Hz, 1H), 7.51 (t, J = 7.9 Hz, 1H), 7.38 (s, 2H) ppm.

### (3) Synthesis of N-(5-(3-bromophenyl)-1,3,4-oxadiazol-2-yl)-3fluoro-5-(trifluoromethyl)benzamide (31 mg, 34% yield, white solid):

To a stirred solution of 5-(3-bromophenyl)-1,3,4-oxadiazol-2-amine (50 mg, 0.21 mmol) in pyridine (3 mL), 3-fluoro-5-(trifluoromethyl)benzoylchloride (0.05 ml, 0.32 mmol) was added. The reaction mixture was stirred at 73°C for 14 hours and then cooled to room temperature. To the mixture, 10% hydrochloric acid (10 ml) and water (10 ml) were added, followed by extraction with ethyl acetate (20 mL) (x3). The combined organic layers were dried over anhydrous MgSO₄, filtered, and concentrated in vacuo. The residue was purified by filtration using DCM and n-Hexane to obtain the product.

¹H NMR (500 MHz, DMSO-*d*₆) : δ_{H} 12.84 (s, 1H), 8.26 (s, 1H), 8.15 (d, *J* = 8.9 Hz, 1H), 8.05 (d, *J* = 10.5 Hz, 2H), 7.97 (d, *J* = 7.8 Hz, 1H), 7.85 (d, *J* = 8.2 Hz, 1H), 7.59 (t, *J* = 7.9 Hz, 1H). ppm HRMS (EI) *m*/*z* [M]⁺ calcd for C₁₆H₉BrF₄N₃O₂, 430.0, found 386.0.

### Experimental Example 1. Lucigenin chemiluminescence assay experiment

### Experimental Methods

Human NOX isozymes were provided from transgenic flies expressing human NOX isozymes from the daughterless (Da)-GAL4 promoter. The genotype of each fly line is shown below.
- human NOX2 : UAS-hNOX2/UAS-dDuox-RNAi; Da-GAL4/+
- human NOX4 : UAS-hNOX4/UAS-dDuox-RNAi; Da-GAL4/+

Transgenic flies were homogenized in PBS containing protease inhibitors (aprotinin, leupeptin) to obtain drosophila membranes of each NOX isozyme. The membranes were incubated with the compounds (100 µM, 10 µM, 1 µM, 0.1 µM, 0.01 µM, 0.001 µM, and 0 µM) for 10 minutes in a shaker. Then, to the membranes, a mixture of 500 µM NADPH (β-nicotinamide adenine dinucleotide 2'-phosphate reduced tetrasodium salt hydrate; Sigma-Aldrich) and 400 µM lucigenin (N,N'-dimethyl-9,9'-biacridinium dinitrate; Sigma-Aldrich) in 1X HEPES (4-(2-hydroxyethyl)-1-piperazineethanesulfonic acid) buffer was added. Lucigenin chemiluminescence was detected at 1-minute intervals for 10 minutes using a multimode microplate reader (SpectraMax iD3, Molecular Devices). IC₅₀ values were calculated with GraphPad Prism 5 (GraphPad Software).

### (1) Results of measuring IC₅₀ values of NOX inhibiting compound

The lucigenin chemiluminescence assay was performed with drosophila membranes expressing hNOX1, hNOX2, and hNOX4. The assay was repeated three times and the IC₅₀ values were averaged.

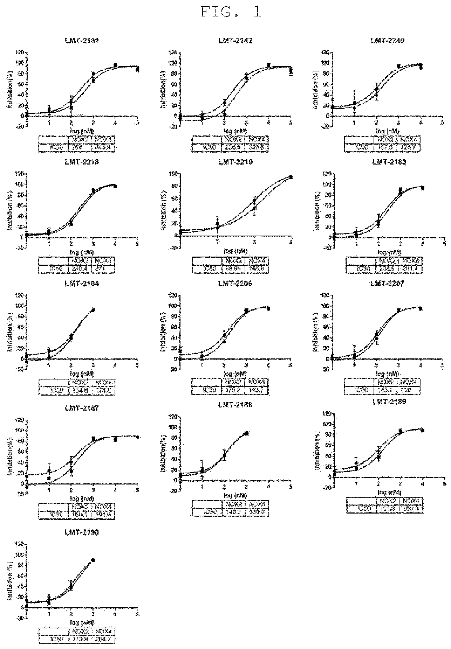

Table 1 and FIG. 1 below show the results of determining the NOX2 and NOX4 inhibitory effects of Examples 1 to 13 according to The present disclosure.

**[Table 1]**

| **IC₅₀ (µM)** | **NOX2** | **NOX4** |
|---|---|---|
| **Example 1 (LMT-2219)** | 0.09 | 0.16 |
| **Example 2 (LMT-2131)** | 0.26 | 0.44 |
| **Example 3 (LMT-2142)** | 0.24 | 0.38 |
| **Example 4 (LMT-2183)** | 0.21 | 0.25 |
| **Example 5 (LMT-2206)** | 0.18 | 0.14 |
| **Example 6 (LMT-2218)** | 0.23 | 0.27 |
| **Example 7 (LMT-2240)** | 0.19 | 0.12 |
| **Example 8 (LMT-2187)** | 0.16 | 0.19 |
| **Example 9 (LMT-2188)** | 0.15 | 0.13 |
| **Example 10 (LMT-2189)** | 0.10 | 0.16 |
| **Example 11 (LMT-2190)** | 0.17 | 0.26 |
| **Example 12 (LMT-2207)** | 0.14 | 0.12 |
| **Example 13 (LMT-2184)** | 0.15 | 0.17 |

### (2) Analysis results of the NOX inhibitory effect of the compound of Example 1 and the compound of Example 2 in BV2 cells

BV2 cells, a mouse microglial cell line, were washed with Hank's balanced salt solution (HBSS) and stabilized by incubation at 37□ in HBSS for 30 minutes. Then, the compounds (compound of Example 1 or compound of Example 2) were treated at various concentrations (0 nM, 1 nM, 10 nM, 100 nM, 1000 nM, and 10000 nM). After 30 minutes, 10 ng/ml of lipopolysaccharide (LPS) and 200 µM of lucigenin were added to the cells. Lucigenin chemiluminescence induced by active oxygen (reactive oxygen species) was detected at 10-second intervals for 15 minutes using a luminometer (GLOMA, Promega).

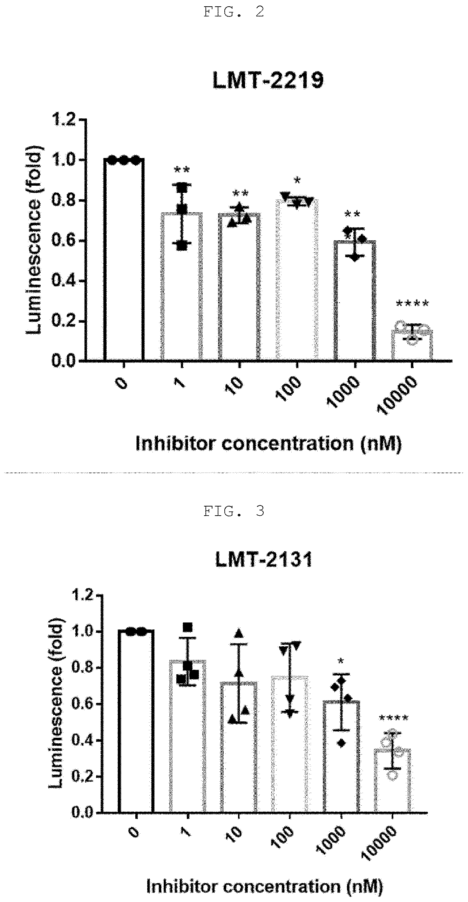

The results are shown in FIGS. 2 and 3.

As shown in FIGS. 2 and 3, BV2 cells were pre-treated with the compounds according to The present disclosure (Example 1 or Example 2) for 30 minutes and the production of active oxygen by stimulation with LPS (10 ng/ml) was determined. Then, it was found that the production of reactive oxygen species was significantly reduced as the treatment concentration of the compounds increased. In other words, the compounds effectively inhibited NOX in microglia and suppressed the production of reactive oxygen species.

### Experimental Example 2. Cytotoxicity experiment (MTT assay)

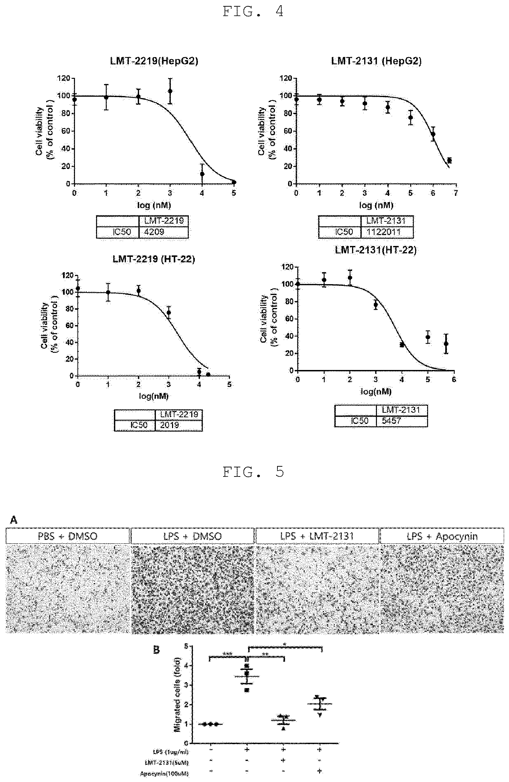

HepG2 (human hepatocellular carcinoma) and HT-22 (mouse hippocampal neuronal cell line) were seeded at densities of 1 × 10⁵ or 1.5 × 10⁴ per well in a 48-well plate and incubated for at least 12 hours, then treated with compounds of Example 1 and Example 2 at various concentrations (0 nM, 1 nM, 10 nM, 100 nM, 1000 nM, 10000 nM, 100000 nM, and 1000000 nM) and incubated for 24 hours. MTT solution (Thiazolyl Blue Tetrazolium Bromide 2 mg/ml) was subsequently added at 30 µl per well. After incubation for 4 hours, the culture medium containing the MTT solution was removed and 300 µl of DMSO was added to dissolve the MTT-formazan. 100 ul of the solution in each well was transferred to a 96-well plate and the absorbance was measured at 570 nm. IC₅₀ values were calculated with GraphPad Prism 5 (GraphPad Software). The cell viability of the groups treated with the compounds of Example 1 and Example 2 at each concentration was determined relative to control group in which HepG2 cells were treated with DMSO alone, in at least five replicates. Results thereof are shown in FIG. 4.

As shown in FIG. 4, in HepG2, Example 1 had the IC₅₀ value of 4.2 µM and Example 2 had the IC₅₀ value of 1.1 mM. In HT-22, Example 1 had the IC₅₀ value of 2.0 µM and Example 2 had the IC₅₀ value of 5.5 µM.

### Experimental Example 3. Inhibitory effect of microglia (BV2) migration

To determine the migration level of BV2 cells, 8 µm-sized transwells (35224, SPL) were used. 1 × 10⁵ BV2 cells were seeded into the insertion transwells, and the lower wells were treated with the compound of Example 2 or Apocynin (NOX inhibitor) along with lipopolysaccharide (LPS, 1 µg/ml) as stimulus. After 24 hours of incubation, BV2 cells that migrated to the bottom of the insertion transwells were stained with crystal violet.

Results thereof are shown in FIG. 5. FIG. 5A is a representative image of each experimental group and FIG. 5B shows the number of migrated BV2 cells confirmed compared to the number of cells treated with PBS, the control.

As shown in FIG. 5, as to the treatment with the compound of Example 2, LPS (1 µg/ml) inhibited the migration of BV2 cells, a mouse microglial cell line. LPS increased the number of migrated BV2 cells by 3.5-fold (*** p<0.001), but this increase was offset by the treatment of the compound of Example 2 (10 µM) (** p<0.01). The offset of BV2 cell migration by oxadiazole derivative compounds is more effective than Apocynin (* p<0.05), a known NOX inhibitor.

### Experimental Example 4. Confirmation of therapeutic effects in Parkinson's animal model

### (1) Experimental Methods

### 1) Efficacy assay of compounds against MPTP-induced Parkinson's disease

To evaluate the effect of compounds of Example 1 and Example 2 on MPTP (1-methyl-4-phenyl-1,2,3,6-tetrahydropyridine hydrochloride; Sigma-Aldrich)-induced Parkinson's disease, C57BL/6 male mice (9 weeks old) were randomly divided into three groups.
- saline + vehicle
- MPTP + vehicle
- MPTP + compound of Example 1 or Example 3 (30 mg/kg)

To induce Parkinson's disease, mice were intraperitoneally injected with MPTP (25 mg/kg) four times at 2-hour intervals, while control mice were treated with an equal volume of saline. The compounds of Example 1 or Example 2 were dissolved in a solvent consisting of 10% DMAC (dimethyl acetamide, Sigma-Aldrich), 10% Tween 80 (Sigma-Aldrich) and 80% saline (JW Pharmaceuticals). Mice were orally administered with 30 mg/kg of the compound of Example 1 or Example 2 starting 1 day prior to MPTP injection for 8 days.

### 2) Immunohistochemical staining experiment

Seven days after MPTP administration (one day after the last administration of the compound of Example 1 or Example 2), experimental animals were sacrificed by perfusion with saline (JW Pharmaceuticals) followed by 10% formalin (Sigma-Aldrich), and brain tissues were obtained. The tissues were cut into 30 um thick slices using a cryostat (Leica) and stored in an anti-freezing solution (30% sucrose, 1% polyvinylpyrrolidone, and 30% ethylene glycol in 1X PBS) at -20°C.

Anti-tyrosine hydroxylase antibody (1:2000-4000, ABCAOM) was used to detect dopaminergic neurons and anti-Iba1 antibody (1:1000, Wako) was used to measure microglia. Biotinylated goat anti-rabbit antibody (VECTOR, BA-1000) was used as a secondary antibody and ABC solution (VECTOR, BA-1000) was used. The immunoreactive sections were then incubated with 3-3' diaminobenzidine (DAB, Dako) to change the color to brown.

For the substantia nigra, six coronal sections, one every 150 um, between -2.80 mm and -3.80 mm bregma were used, and for the striatum, three coronal sections between +0.98 mm and +0.50 mm bregma were collected. The coronal sections were stained with anti-tyrosine hydroxylase antibody or anti-Iba1 antibody and then analyzed by counting the number of stained cells or using ImagePro plus 7.0 software.

### 3) Behavioral test (Pole test)

The mice were moved to a home cage where they were placed head up in the top of a 60 cm vertical pole with a diameter of 1 cm, and were then allowed to descend themselves and stay for about 15 seconds in the home cage.

The above procedure was repeated three times, all of which were videotaped, and the time for the mouse's head to turn completely downward (T-turn) and total time to descend to the floor (T-total) were analyzed.

### (2) Experimental results for compounds of Example 1 and Example 2

### 1) Confirmation of dopamine neuronal protection effect

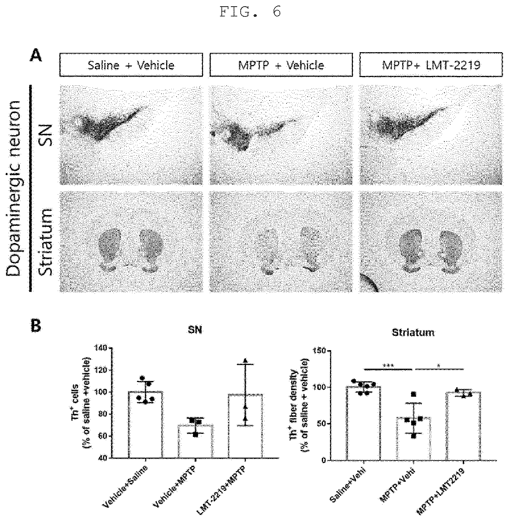

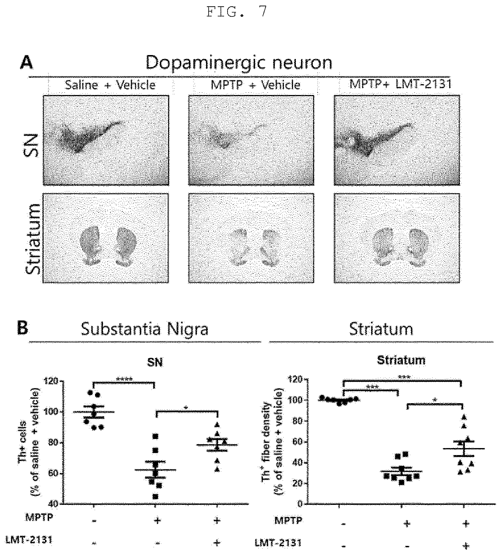

Dopaminergic neurons were identified in the MPTP Parkinson's disease-induced models in the treatment with the compounds of Example 1 or Example 2 according to The present disclosure, and are shown in FIG. 6 (Example 1) and FIG. 7 (Example 2).

In FIGS. 6 and 7, (A) shows representative tyrosine hydroxylase (Th) immunostaining images of the substantia nigra and striatum, and (B) shows the results of counting Th⁺ cells in six sections of the substantia nigra (SN) and measuring Th⁺ fiber density in three sections of the striatum. (Determined by Student's t-test *p < 0.05, *** p < 0.0005, ****p <0.0001).

As can be seen from the above results, the Vehicle-treated group had a decrease in the number of dopamine neurons in the substantia nigra and a decrease in the Th+ fiber density in the striatum due to MPTP. On the other hand, compared to the vehicle-treated group, the group treated with the compounds of Example 1 or Example 2 had a higher number of dopaminergic neurons in the substantia nigra and a higher density of Th⁺ fibers in the striatum.

The above results confirmed that the oxadiazole derivatives according to The present disclosure have excellent effects in protecting dopaminergic neurons.

### 2) Immunohistochemical staining result

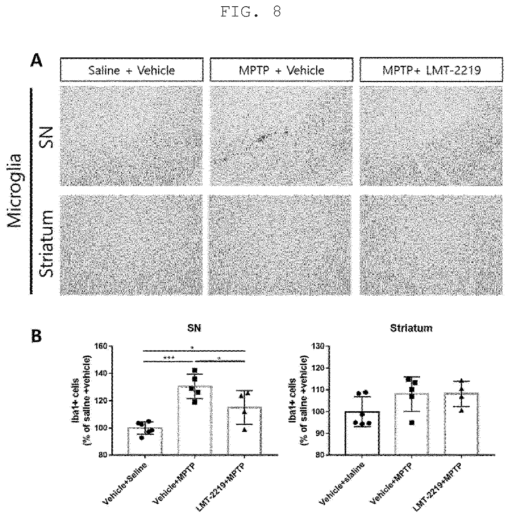

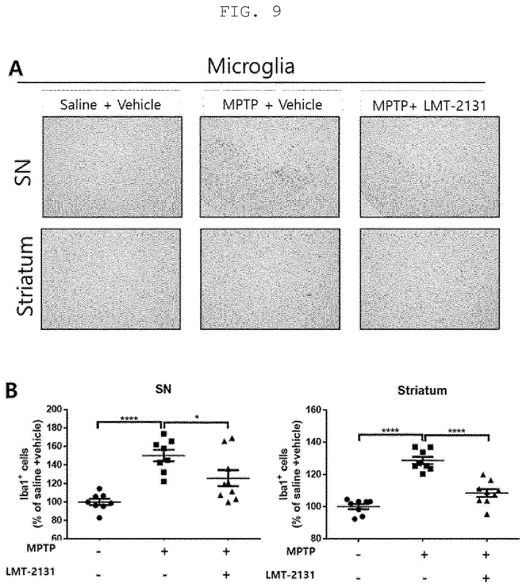

Treatment with the compounds of Example 1 or Example 2 according to The present disclosure was confirmed to ameliorate the microglial pathology in MPTP-induced Parkinson's disease, which is shown in FIG. 8 (Example 1) and FIG. 9 (Example 2).

In FIGS. 8 and 9, (A) shows representative Iba1 immunostained images of the substantia nigra (SN) and striatum, and (B) shows the results of counting Iba1⁺ cells in the substantia nigra (SN) and the striatum. (Determined by Student's t-test * p <0.05, ** p <0.005, *** p <0.0005, ****p <0.0001) .

Specifically, the vehicle-treated group had a significant increase in the number of microglia in the substantia nigra and striatum by MPTP, and this increase in microglia was effectively reduced by treatment with the compounds of Example 1 or Example 2 according to The present disclosure.

The above results confirmed that the oxadiazole derivatives according to The present disclosure have excellent effects in ameliorating the pathology of microglia.

### 3) Behavioral test (pole test) results

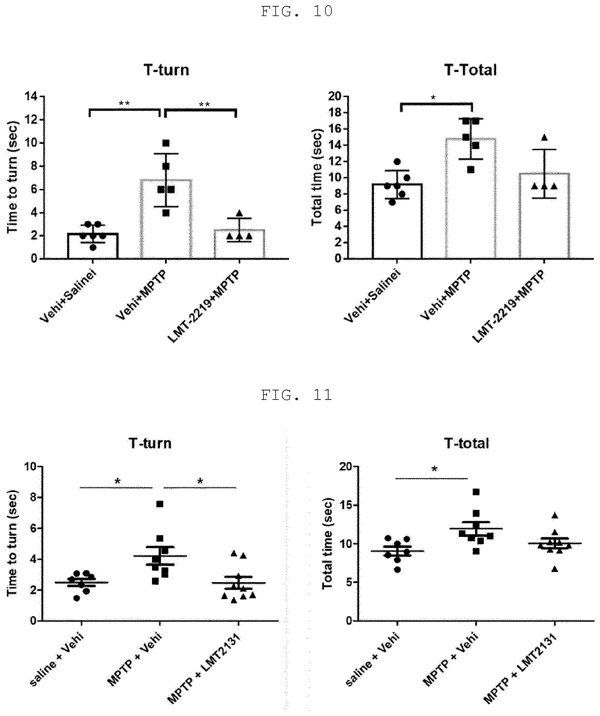

Treatment with the compounds of Example 1 or Example 2 according to The present disclosure was also confirmed to improve behavioral characteristics in MPTP-induced Parkinson's disease, which is shown in FIGS. 10 and 11.

As shown in FIGS. 10 and 11, when a behavioral test (pole test) was performed on Day 6 after MPTP administration, the vehicle-treated group showed a significant increase in T-turn and T-time induced by MPTP (* p < 0.05, ** p < 0.005), but the group administered with the compounds of Example 1 or Example 2 according to The present disclosure resulted in a decrease in T-turn (* p < 0.05, ** p < 0.005) and a significant decrease in T-total (* p < 0.05, ** p < 0.005) compared to the vehicle-treated group.

In other words, the oxadiazole derivatives according to The present disclosure were found to be effective not only in molecular biological improvement, but also in actual behavioral improvement.

### Experimental Example 5. Confirmation of therapeutic effects in Alzheimer's animal model

### (1) Experimental Methods

### 1) Efficacy analysis experiment of compound on Tg-APPswe/PS1dE9 (Tg-APP/PS1) Alzheimer's animal model

The Tg-APP/PS1 animal models were used to determine the effect of compounds of Examples according to The present disclosure on Alzheimer's disease. This animal models show amyloid plaque deposition as early as 6.5 months and severe cognitive impairment at 6 to 8 months. The Tg-APP/PS1 animal models were administered with vehicle or the Example compound (i.e., Example 2 was used) at 30 mg/kg or 10 mg/kg every other day from 6.5 to 8 months. All behavioral testing occurred between 7.5 and 8 months, and brain tissues for biopsy were obtained by perfusion after behavioral testing.

### 2) Confirmation of amyloid plaque reduction (Thioflavin-S staining)

To confirm amyloid beta plaques, the brain tissues obtained in the above part (1)_1) were incubated in 1% Thioflavin-S solution for 8 to 20 minutes and washed with ethanol and distilled water. The stained tissues were sectioned and the number of plaques or stained areas were quantified.

### 3) Confirmation of inhibition of astrocyte and microglia activity (GFAP (astrocytes), Iba1 (microglia) immunostaining)

To confirm the changes in astrocytes and microglia by administration of oxadiazole derivative compounds, immunostaining was performed using GFAP antibody, a marker for astrocytes, and Iba1 antibody, a marker for microglia.

### 4) Confirmation of cognitive improvement (Novel object recognition-NOR, Novel location recognition-NLR)

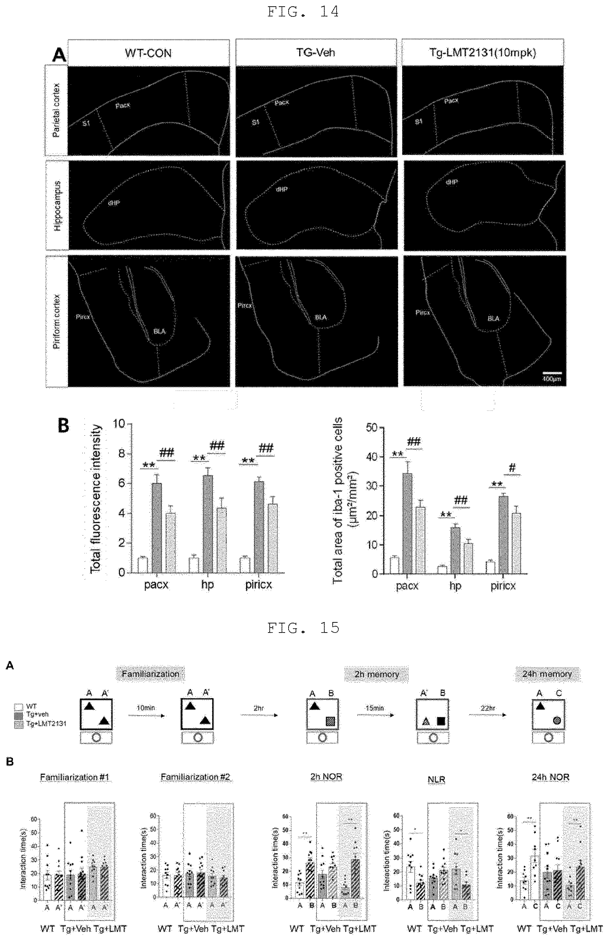

The novel object recognition (NOR) and novel location recognition (NLR) tests are behavioral assessments that evaluate memory based on the disparity in search time between a novel object or an object relocated to a new position compared to a familiar object. A schematic diagram of the experimental method is shown in FIG. 15A. Each experimental animal was placed in a square box with two identical objects and allowed to familiarize for 10 minutes (familiarization#1) . Ten minutes after the first familiarization was completed, the objects used in the first familiarization were returned to their original position and subjected to a second familiarization (familiarization#2). After 2 hours, only one of the objects used in familiarization was replaced with a novel object and placed in the same position and the exploration time of the familiar and novel objects (2h NOR) was determined. After 15 minutes, the familiar object was repositioned, and the exploration time of both objects was checked (NLR). After 22 hours (24 hours after familiarization), the novel object and the familiar object were placed and the exploration time was determined (24h NOR).

### 5) Confirmation of spatial memory improvement (water maze test)

Water maze test is frequently used to assess memory functions such as memory formation, consolidation, and retrieval effects by assessing the time it takes for an experimental animal to find a platform beneath the water surface, relying on environmental cues. Therefore, the treatment effect on Alzheimer's disease was confirmed using the model used in the water maze test above.

### 6) Confirmation of improvement in short-term and long-term memory of fear memory (passive avoidance test)

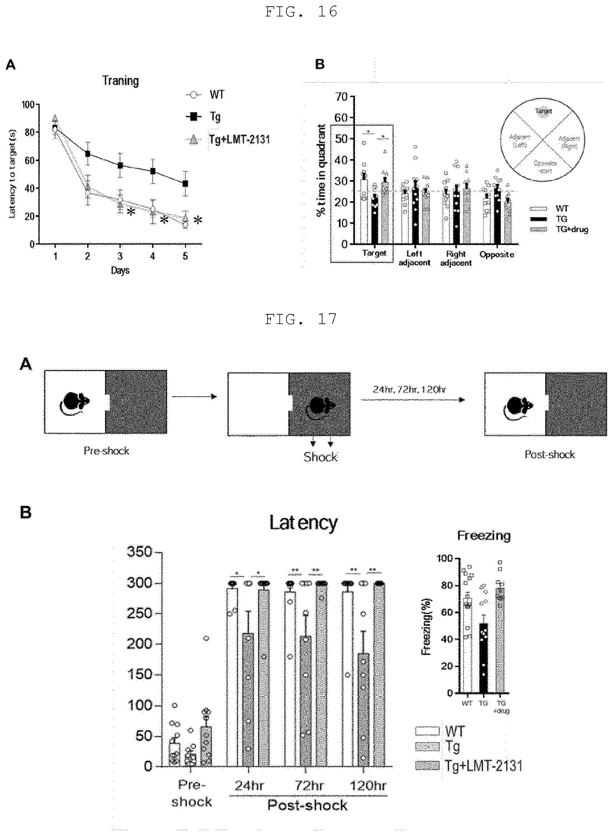

The passive avoidance test is a fear-motivated test mainly used to evaluate short-term or long-term memory in experimental animals. A schematic diagram of the experimental method is shown in FIG. 17A. The passive avoidance test evaluates fear memory by utilizing the innate tendency of experimental animals to prefer dark areas and avoid bright areas, delivering an electric shock to the paw upon entry into a dark area, and measuring the time it takes for the animal to re-enter the dark area upon re-exposure to the same environment.

### (2) Experimental results for compound of Example 2

### 1) Confirmation of amyloid plaque reduction

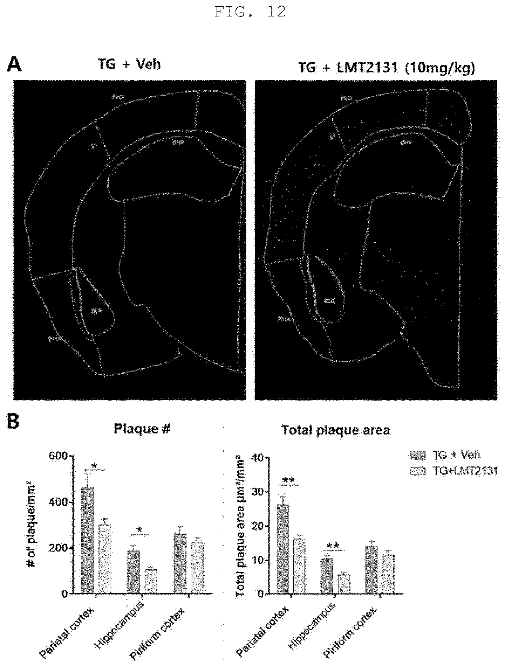

The alleviation of amyloid plaque disease was confirmed using the compound of Example 2 of the oxadiazole derivatives according to The present disclosure, which is shown in FIG. 12. In FIG. 12, (A) shows representative images of Thioflavin-S staining of regions including the parietal cortex, piriform cortex, and hippocampus of the vehicle-treated group (TG+Veh) and the compound of Example 2 (TG+LMT2131) in Tg-APP/PS1 mice, and (B) shows the quantification of the number of plaques and stained areas in the parietal cortex and hippocampus by analyzing the images.

As shown in FIG. 12 above, treatment with the compounds according to The present disclosure demonstrated a highly significant ameliorative effect on amyloid plaque deposition, which is one of the primary symptoms of Alzheimer's disease.

### 2) Confirmation of inhibition of astrocyte and microglial activity

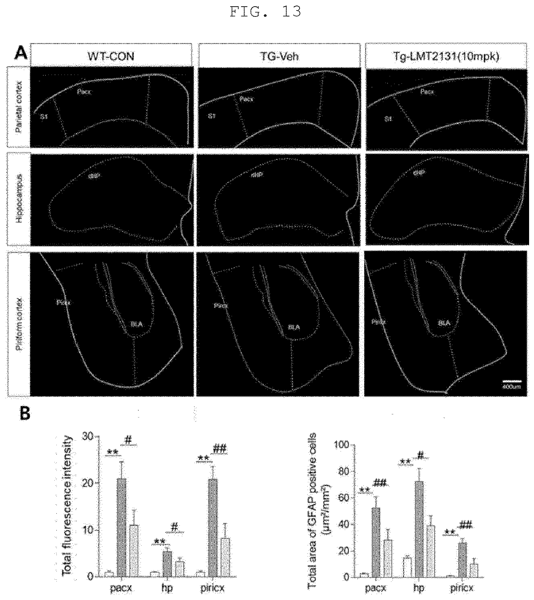

FIGS. 13 and 14 show the results of inhibition of astrocyte and microglia activity.

In FIGS. 13 and 14, (A) shows representative images of parietal cortex, piriform cortex, and hippocampus from each group and (B) shows the quantification of GFAP immunostained fluorescence intensity and area.

First, as shown in FIG. 13, treatment with compounds according to The present disclosure ameliorated astrocyte pathology. Tg-APP/PS1 mice exhibited significantly increased activation of astrocytes in all of the parietal cortex, piriform cortex, and hippocampus, as compared to wild-type controls. On the other hand, the activation of astrocytes was effectively reduced in all of the parietal cortex, piriform cortex, and hippocampus by the administration of the compounds according to The present disclosure.

First, as shown in FIG. 14, treatment with compounds according to The present disclosure ameliorated microglial pathology. Tg-APP/PS1 mice exhibited significantly increased activation of microglia in all of the parietal cortex, piriform cortex, and hippocampus, as compared to wild-type controls. On the other hand, the activation of microglia was effectively reduced in all of the parietal cortex, piriform cortex, and hippocampus by the administration of the compounds according to The present disclosure.

The above results confirmed that the compounds according to The present disclosure are effective in reducing inflammation in nerves and inhibiting astrocyte and microglial activity.

### 3) Confirmation of cognitive improvement

The results of the object recognition tests to confirm cognitive improvements are shown in FIG. 15.

It was confirmed that the familiarization process allowed all groups to explore without any preference for a particular object or location (familiarization). As a result of the novel object recognition test after 2 hours (2h NOR), the Tg-APP/PS1 Alzheimer's mouse models showed no significant difference in the time taken to explore novel and familiar objects, but the Tg-APP/PS1 Alzheimer's mouse models administered with 30 mg/kg of the compound according to The present disclosure exhibited a significantly increase in exploration time for novel objects than familiar objects, comparable to wild-type animals. As a result of the novel location recognition (NLR) test, the Tg-APP/PS1 Alzheimer's mouse models showed no difference in the time taken to explore objects placed in a novel location versus objects placed in a familiar location, but the Tg-APP/PS1 mice administered with 30 mg/kg of the compound according to The present disclosure exhibited a significant increase in exploration time for objects located in a novel location. As a result in a novel localization recognition test performed 24 hours later (24 h NOR), the vehicle-administered Tg-APP/PS1 mice showed no difference in exploration time between familiar and novel objects. On the other hand, Tg-APP/PS1 mice administered with 30 mg/kg of the compound according to The present disclosure exhibited a remarkable increase in exploration time for novel objects than familiar objects, which was similar to that observed in wild-type animals.

The above results confirmed that the oxadiazole derivatives according to The present disclosure improve the recognition of novel objects and locations and have excellent effects on improvement in short-term as well as long-term memory.

### 4) Confirmation of improvement in spatial memory

The results of the water maze test to confirm improvement in spatial memory are shown in FIG. 16.

FIG. 16(A) shows the time required to locate a subsurface fixed platform on five consecutive days. All animals required approximately 80 seconds to locate the platform on the first day, and over time, wild-type (WT) animals showed a decrease in the time required to locate the platform. The vehicle-administered Tg-APP/PS1 mice had difficulty in learning the location of the platform, exhibiting significantly longer times to locate the platform compared to wild-type mice over time. On the other hand, Tg-APP/PS1 mice administered with the compounds according to The present disclosure demonstrated memory acquisition ability equivalent to wild-type mice.

After 5 days of training, the platform was removed from under the water surface 24 hours later and the time spent in the quadrant where the platform was originally located was checked to determine the extent to which the mice remembered the acquired platform location, as shown in FIG. 16(B). The results showed that wild-type mice stayed longer in the quadrant where the platform was located compared to other quadrants, while the vehicle-administered Tg-APP/PS1 mice spent significantly less time in the corresponding quadrant compared to wild-type. The Tg-APP/PS1 mice administered with the compounds according to The present disclosure stayed in the corresponding quadrant to locate the platform, similar to wild type mice.

The above results thus confirmed that the oxadiazole derivatives according to The present disclosure have excellent effects on the formation, consolidation and retrieval of spatial memory.

### 5) Confirmation of improvements in short-term and long-term memory of fear memories

The results of the passive avoidance test to confirm for short-term and long-term memory improvement are shown in FIG. 17.

As shown in FIG. 17(B), prior to being subjected to the electric shock (pre-shock), all experimental animals exhibited reduced movement time from the light to the dark area. However, after the electric shock (post-shock), the majority of wild-type animals refrained from entering the dark area where they had previously received a shock for the duration of the experiment, which lasted 300 seconds, the end time of the experiment, and this fear memory was retained 120 hours later (286.36 seconds). Meanwhile, the vehicle-administered Tg-APP/PS1 mice had a significantly shorter time to enter the dark zone than wild-type, at 217.89 seconds, starting 24 hours after receiving the electric shock. The time decreased over time, and after 120 hours, the mice entered the dark area where they were electroshocked an average of 185 seconds later.

In contrast, Tg-APP/PS1 mice administered with the compounds according to The present disclosure exhibited fear memory retention comparable to that of wild-type animals. This behavior was similarly observed in the freezing time induced by fear memory.

These results confirmed that the oxadiazole derivatives according to The present disclosure have a highly favorable impact on short-term and long-term memory.

### Experimental Example 6. Confirmation of glioblastoma growth inhibition

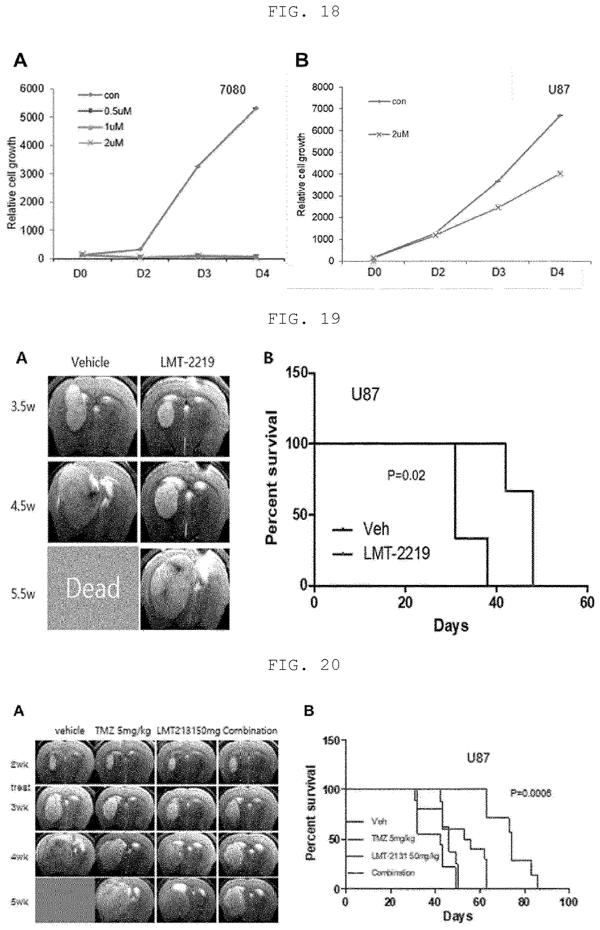

Cell growth experiments were performed to determine the effect of the compounds according to The present disclosure on the growth of brain tumor glioblast cells. Specifically, the rodent brain tumor glioblast cell line 7080 and the human glioblast cell line U87 were treated with the compound of Example 2 according to The present disclosure to determine the cell growth inhibitory effects, and the results are shown in FIG. 18.

As shown in FIG. 18, the glioma cell growth of glioblast cell line 7080 was strongly inhibited at all concentrations (0.5 um, 1 µM, and 2 µM). Similarly, as to the human glioblast cell line U87, cell growth inhibition was observed starting 4 days after treatment with 2 µM of the compound of The present disclosure.

### Experimental Example 7. Confirmation of anti-cancer effects in mouse animal model xenografted with brain tumor glioblastoma

To confirm the therapeutic effect on brain tumor glioblastoma, U87 (5 × 10⁴), a human brain tumor cell line, was xenografted into the brains of mice to establish a brain tumor model, followed by oral administration of the drugs.

Different experimental groups were evaluated for each compound.

First, with respect to the compound of Example 1, the experimental groups were divided into Vehicle (n=3) and the compound of Example 1, 50 mg/kg (n=3) and the administered with the drug orally 5 times a week 20 days after U87 xenografting.

With respect to the compound of Example 2, the experimental groups were divided into Vehicle (n=9), TMZ 5 mg/kg (Temozolomide, n=10, 2 times per week), the compound of Example 2, 50 mg/kg (n=8, 5 times per week), and combination (TMZ + the compound of Example 2, n=7), and began receiving the drug 20 days after U87 xenografting.

The results of each experiment are shown in FIGS. 19 and 20.

FIG. 19 shows the results of treatment with the compound of Example 1. FIG. 19(A) shows representative MRI images at 3.5 weeks, 4.5 weeks, and 5.5 weeks after U87 xenografting for each group, and 19(B) shows the survival rate results. The median survival for the vehicle-treated group was 31 days, whereas the group treated with the compound of Example 1 had a median survival of 48 days, representing a significant extension of 17 days compared to the vehicle group.

FIG. 20 also shows the results of administering the compound of Example 2. As shown in FIG. 20, representative MRI images at 2, 3, 4, and 5 weeks after U87 xenografting for each group show that brain tumor sizes are similar for all groups at 2 weeks after glioblastoma implantation (before drug administration).

Tumor size was the largest in the vehicle-administered group, and the administration of TMZ alone or the compound of Example 2 alone inhibited the tumor growth rate. In particular, the group administered with the combination of TMZ and the compound of Example 2 inhibited the tumor growth rate the most.

Specifically, the vehicle-administered group had the shortest median survival time (42 days), followed by Example 2 alone (46 days), TMZ alone (54.5 days), and the combination (74 days), with the combination group having 32 days longer survival time than the vehicle group.

The results of experiments on the xenograft mouse brain tumor model confirmed that the compounds according to The present disclosure exhibit excellent anti-cancer effects, and in particular, the compounds exhibit significant synergistic effects on the treatment of glioblastoma by coadministration with other anti-cancer drugs such as temozolomide.

### Experimental Example 8. Confirmation of brain barrier blood permeability with PAMPA-BBB assay

The parallel artificial membrane permeability assay (PAMPA) was performed to determine the brain barrier blood (BBB) permeability.

Specifically, deep well plates were prepared by dispensing donor buffer at pH 7.4 and mixing the test compounds (Example 1, 2, 10, 12, and 13 compounds; control compounds Progesterone, Lidocaine, and Ranitidine). For the Blank measurement, the donor buffer was dispensed into a high sensitivity UV plate at 150 µl per well and the UV values were measured (Blank sample). For the initial value measurement, the mixture in the deep well plate was dispensed 150 µl per well into the UV plate and the UV values were measured (initial sample). Next, 200 µl of the test compound mixture was dispensed onto the donor plate of the PAMPA sandwich, and the acceptor plate of the PAMPA sandwich was inverted and the membrane was coated with 5 µl of BBB-lipid solution. Then, 200 µl of acceptor sink buffer was added to each well of the acceptor plate, and the acceptor plate and donor plate were combined and incubated at 25□ for 4 hours. After 4 hours of incubation, the acceptor plate and donor plate were separated and 150 µl each was transferred to the UV plate to measure the UV values (donor sample, and acceptor sample). Finally, the permeability (Pₑ(10⁻⁶cm/sec)) was analyzed using the PAMPA explore program. The experiment was performed in three replicates.

The permeability of the material was analyzed by UV measurement and LC-MS/MS, the specific conditions of which are described below.

### UV Analysis

**[Table 2]**

| | |
|---|---|
| **Software** | PION PAMPA EXPLORER (Version 3.8) |
| **Protocol** | BBB protocol (pH 7.4) |
| **Wave length analyzed** | 250-498 nm |
| **Temperature** | 25°C |
| **Incubation time** | 4 hrs |
| **Calculation basis** | acceptor+donor+membrane; double sink |
| **Parameters** | sample+impurity+background |
| **Detection** | threshold 0.015 |
| **Permeability classification** | high (>0.4x10⁻⁶), low (<0.4x10⁻⁶) |

In LC-MS/MS, if the spectra of the Reference (Initial), Donor, and Acceptor samples were found to be very low or not measurable during U.V absorbance measurement, the sample concentration was reanalyzed using LC-MS/MS.

The Pₑ (10⁻⁶ cm/sec) value of 0.4 was used as a benchmark to categorize the permeability as high if it is higher and low if it is lower.

The results of the analysis of Pₑ(10⁻⁶cm/sec) values for Examples 1, 10, 12, and 13 are shown in Table 3 below.

**[Table 3]**

| Compound | Concentration | Incubation time | Permeability Pₑ(10⁻⁶cm/sec) | Method |
|---|---|---|---|---|
| Progesterone | 50 µM | 4 hr | High | U.V |
| Ranitidine | 50 µM | 4 hr | Low | U.V |
| Example 1 | 50 µM | 4 hr | High | U.V |
| Example 10 | 50 µM | 4 hr | High | U.V |
| Example 11 | 50 µM | 4 hr | High | U.V |
| Example 13 | 50 µM | 4 hr | High | U.V |

In addition, BBB permeability was confirmed with respect to the compound of Example 2 at a concentration of 10 µM.

**[Table 4]**

| Compound | Concentration | Incubation time | Permeability Pₑ(10⁻⁶cm/sec) | Method |
|---|---|---|---|---|
| Progesterone | 50 µM | 4 hr | High | U.V |
| Ranitidine | 50 µM | 4 hr | Low | U.V |
| Lidocaine | 50 µM | 4 hr | High | U.V |
| Example 2 | 10 µM | 4 hr | High | U.V |

As can be seen above, the oxadiazole derivatives according to The present disclosure have excellent BBB permeability, showing a high potential for application in diseases where BBB permeability is an essential consideration, such as degenerative brain diseases and brain cancer.

### Experimental Example 9. Verification of metabolic stability

The stability of a drug may be predicted by using liver microsomes to determine the extent to which the drug is metabolized in the liver. The metabolic stability is a factor capable of affecting PK parameters such as drug clearance, half-life, and oral bioavailability, and is an important characteristic for a potential drug candidate. Thus, a metabolic stability verification experiment was performed on the oxadiazole derivatives according to The present disclosure.

Specifically, sample treatment human liver microsomes (0.5 mg/ml), 0.1 M phosphate buffer (pH 7.4), and test compound at a concentration of 1 µM were added, and the mixture was pre-incubated at 37°C for 5 minutes, followed by addition of NADPH Regeneration system solution and incubation at 37°C for 30 minutes. An acetonitrile solution containing an internal standard (chlorpropamide) was then added to terminate the reaction, followed by centrifugation for 5 minutes (14,000 rpm, 4°C). Then, the supernatant was injected into the LC-MS/MS system to analyze the substrate drug to evaluate the metabolic stability of the two compounds.

For LC-MS/MS analysis, the amount of substrate remaining from the above reaction was analyzed using an Agilent 1290 infinity series pump system (Agilent, USA) and a Triple Quad 5500 LC-MS/MS system (Applied Biosystems, USA) . The HPLC column was an Acquity UPLC BEH Amide column (2.1 × 100 mm, 1.7 um particle size; Waters, Ireland). The mobile phases were distilled water containing 0.1% formic acid (A) and acetonitrile containing 0.1% formic acid (B). Data analysis was performed using Analyst software (version 1.6.3).

The evaluation criteria were set as follows.

### Evaluation criteria for microsomal stability based on % remaining, which is the result of 30-minute reaction experiment

> 90% : considered as a highly stable compound with a half-life of 3 hours or longer
70-90% : considered as a stable compound with a half-life of 1-3 hours
50-70% : considered as a relatively stable compound with a half-life of 30 to 60 minutes
30-50% : considered as a relatively unstable compound with a half-life of 15 to 30 minutes
Less than 30%: considered as a compound with a half-life of 15 minutes or less and expected to be metabolized rapidly

The results of the above experiment are shown in Table 5 and Table 6 below.

**[Table 5]**

| Compound | Human (%) |
|---|---|
| Example 2 | >100 |
| Verapamil (Reference) | 13.1 |

**[Table 6]**

| Compound | Human (%) |
|---|---|
| Example 1 | 81.7 |
| Example 10 | 94.9 |
| Example 12 | 90.0 |
| Example 13 | 79.0 |
| Verapamil (Reference) | 11.9 |

As confirmed above, the oxadiazole derivatives according to The present disclosure exhibited good metabolic stability.

As described above, The present disclosure has been described through Examples. From the above description, those skilled in the art to which The present disclosure pertains will understand that The present disclosure may be embodied in other specific forms without changing the technical spirit or essential characteristics thereof. Therefore, the above-described Examples should be understood as illustrative in all aspects and not limiting. As the scope of The present disclosure, it should be construed that all changes or modifications derived from the meaning and scope of the claims to be described below and equivalents thereof rather than the above detailed description are included in the scope of The present disclosure.

## Claims

1. A compound represented by the following Chemical Formula 1 or a pharmaceutically acceptable salt thereof: 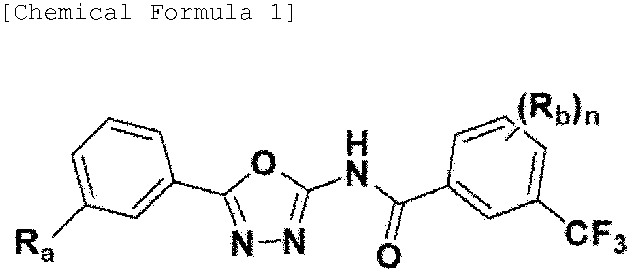
in Chemical Formula 1 above,
Rₐ is hydrogen, halogen, -CF₃, or a 5-6 membered heteroaryl containing at least one heteroatom selected from O, S, and N,
R_{b} is a halogen, and
n is any integer of 1 or 2.

2. The compound of claim 1, wherein the compound represented by Chemical Formula 1 is a compound represented by the following Chemical Formula 3-1: 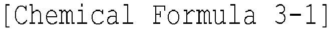 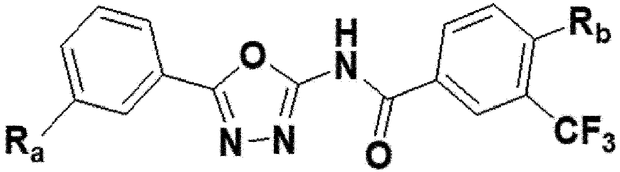
in Chemical Formula 3-1 above,
Rₐ is hydrogen, halogen, -CF₃, or a 5-6 membered heteroaryl containing at least one heteroatom selected from O, S, and N, and
R_{b} is a halogen.

3. The compound of claim 1, wherein the compound represented by Chemical Formula 1 is a compound represented by the following Chemical Formula 3-2: 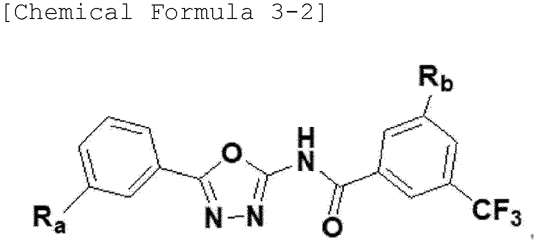
in Chemical Formula 3-2 above,
Rₐ is hydrogen, halogen, -CF₃, or a 5-6 membered heteroaryl containing at least one heteroatom selected from O, S, and N, and
R_{b} is a halogen.

4. The compound of claim 1, wherein Rₐ is any one selected from the group consisting of Cl, Br, CF₃, furanyl, thienyl, pyrrolyl, parazinyl, pyridazinyl, imidazolyl, pyrazolyl, pyrimidinyl, oxazolyl, and pyridinyl,
R_{b} is F, and
n is 1.

5. The compound of claim 4, wherein Rₐ is any one selected from the group consisting of Cl, Br, CF₃, furanyl, and thienyl.

6. The compound of claim 1, wherein Rₐ is hydrogen, R_{b} is F or I, and n is 1.

7. The compound of claim 2, wherein Rₐ is any one selected from the group consisting of hydrogen, Br, Cl, -CF₃, furanyl, thienyl, pyrrolyl, parazinyl, pyridazinyl, imidazolyl, pyrazolyl, pyrimidinyl, oxazolyl, and pyridinyl, and
R_{b} is F or I.

8. The compound of claim 7, wherein Rₐ is any one selected from the group consisting of Cl, Br, CF₃, furanyl, and thienyl.

9. The compound of claim 3, wherein Rₐ is hydrogen or Cl, and R_{b} is F.

10. The compound of claim 1, wherein the compound represented by Chemical Formula 1 is any one selected from the group consisting of the following compounds:
*N*-(5-(3-chlorophenyl)-1,3,4-oxadiazol-2-yl)-3-fluoro-5-(trifluoromethyl)benzamide;
4-fluoro-*N*-(5-phenyl-1,3,4-oxadiazol-2-yl)-3-(trifluoromethyl)benzamide;
3-fluoro-*N*-(5-phenyl-1,3,4-oxadiazol-2-yl)-5-(trifluoromethyl)benzamide;
*N*-(5-(3-bromophenyl)-1,3,4-oxadiazol-2-yl)-4-fluoro-3-(trifluoromethyl)benzamide;
4-fluoro-3-(trifluoromethyl)-*N*-(5-(3-(trifluoromethyl)phenyl)-1,3,4-oxadiazol-2-yl)benzamide;
*N*-(5-(3-chlorophenyl)-1,3,4-oxadiazol-2-yl)-4-fluoro-3-(trifluoromethyl)benzamide;
4-iodo-*N*-(5-phenyl-1,3,4-oxadiazol-2-yl)-3-(trifluoromethyl)benzamide;
4-fluoro-*N*-(5-(3-thiophen-3-yl)phenyl)-1,3,4-oxadiazol-2-yl)-3-(trifluoromethyl)benzamide;
3-fluoro-*N*-(5-(3-thiophen-3-yl)phenyl)-1,3,4-oxadiazol-2-yl)-5-(trifluoromethyl)benzamide;
4-fluoro-*N*-(5-(3-furan-3-yl)phenyl)-1,3,4-oxadiazol-2-yl)-3-(trifluoromethyl)benzamide;
3-fluoro-*N*-(5-(3-furan-3-yl)phenyl)-1,3,4-oxadiazol-2-yl)-5-(trifluoromethyl)benzamide;
3-fluoro-5-(trifluoromethyl)-*N*-(5-(3-(trifluoromethyl)phenyl)-1,3,4-oxadiazol-2-yl)benzamide; and
*N*-(5-(3-bromophenyl)-1,3,4-oxadiazol-2-yl)-3-fluoro-5-(trifluoromethyl)benzamide.

11. A pharmaceutical composition for preventing or treating NADPH oxidase (NOX)-related diseases, comprising: the compound according to any one of claims 1 to 10 or a pharmaceutically acceptable salt thereof,
wherein the NADPH oxidase (NOX)-related disease is any one selected from the group consisting of psoriasis, rheumatoid arthritis, osteoarthritis, atherosclerosis, ulcers, liver cirrhosis, glomerulonephritis, diabetic nephropathy, malignant nephrosclerosis, thrombotic microangiopathy, organ transplant rejection, diabetes, hypertension, cardiac hypertrophy, heart failure, restenosis, cancer, autoimmune diseases, inflammatory diseases, degenerative brain diseases, and retinal diseases.

12. The pharmaceutical composition of claim 11, wherein the degenerative brain disease is any one selected from the group consisting of Parkinson's disease, Huntington's disease, Alzheimer's disease, mild cognitive impairment, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

13. The pharmaceutical composition of claim 11, wherein the cancer is any one selected from the group consisting of brain cancer, neuroendocrine cancer, gastric cancer, lung cancer, breast cancer, ovarian cancer, liver cancer, bronchial cancer, nasopharyngeal cancer, laryngeal cancer, pancreatic cancer, bladder cancer, adrenal cancer, colorectal cancer, colon cancer, cervical cancer, prostate cancer, bone cancer, skin cancer, thyroid cancer, parathyroid cancer, and ureter cancer.

14. The pharmaceutical composition of claim 13, wherein the brain cancer is any one selected from the group consisting of astrocytoma, glioblastoma, ependymoma, oligodendroglioma, mixed glioma, brain stem glioma, optic nerve glioma, pituitary adenoma, craniopharyngioma, medulloblastoma, primitive neuroectodermal tumors, pineal tumors, meningioma, schwannoma, metastatic brain tumors, CNS lymphoma, neurofibromatosis, pseudotumor cerebri, and tuberous sclerosis.

15. A pharmaceutical composition for preventing or treating cancer, comprising: the compound according to any one of claims 1 to 10 or a pharmaceutically acceptable salt thereof; and a second anti-cancer agent,
wherein the second anti-cancer agent is at least any one selected from the group consisting of Cyclophosphamide; Melphalan; Docetaxel; Paclitaxel; Nab-paclitaxel; Carboplatin; Cisplatin; Oxaliplatin; Methotrexate; Pemetrexed; Azathioprine; Capecitabine; Fluouracil; Mercaptopurine; Gemcitabine; Bleomycin; Actinomycin; Vincristine; Vinblastine; Vinorelbine; Retinoic acid; Temozolomide; Daunorubicin; Doxorubicin; Irinotecan; and Topotecan.

16. The pharmaceutical composition of claim 15, wherein the second anti-cancer agent is temozolomide.

17. A pharmaceutical composition comprising:
the compound according to any one of claims 1 to 10, or a pharmaceutically acceptable salt thereof; and a pharmaceutically acceptable carrier.

18. A method for treating NADPH oxidase (NOX)-related diseases, comprising: administering, to a subject in need thereof, a therapeutically effective amount of the compound according to any one of claims 1 to 10 or a pharmaceutically acceptable salt thereof.

19. The method of claim 18, wherein the NADPH oxidase (NOX)-related disease is any one selected from the group consisting of psoriasis, rheumatoid arthritis, osteoarthritis, atherosclerosis, ulcers, liver cirrhosis, glomerulonephritis, diabetic nephropathy, malignant nephrosclerosis, thrombotic microangiopathy, organ transplant rejection, diabetes, hypertension, cardiac hypertrophy, heart failure, restenosis, cancer, autoimmune diseases, inflammatory diseases, degenerative brain diseases, and retinal diseases.

20. A method for treating cancer, comprising: administering, to a subject in need thereof, a therapeutically effective amount of the compound according to any one of claims 1 to 10 or a pharmaceutically acceptable salt thereof.

21. Use of the compound according to any one of claims 1 to 10 or a pharmaceutically acceptable salt thereof for the manufacture of medicaments for the treatment of NADPH oxidase (NOX)-related diseases.

22. The use of claim 21, wherein the NADPH oxidase (NOX)-related disease is any one selected from the group consisting of psoriasis, rheumatoid arthritis, osteoarthritis, atherosclerosis, ulcers, liver cirrhosis, glomerulonephritis, diabetic nephropathy, malignant nephrosclerosis, thrombotic microangiopathy, organ transplant rejection, diabetes, hypertension, cardiac hypertrophy, heart failure, restenosis, cancer, autoimmune diseases, inflammatory diseases, degenerative brain diseases, and retinal diseases.

23. Use of the compound according to any one of claims 1 to 10, or a pharmaceutically acceptable salt thereof for the manufacture of a medicament for the treatment of cancer.
